# EUROPEAN PATENT SPECIFICATION

(11) **EP 1 816 998 B1**
(45) Date of publication and mention of the grant of the patent: **18.03.2009**
(21) Application number: 05823823.9
(22) Date of filing: 22.11.2005
(51) Int. Cl.: A61K 6/00

(54) **FORMULATIONS COMPRISING CERAMIDES AND/OR PSEUDOCERAMIDES AND (ALPHA-)BISABOLOL FOR COMBATING SKIN DAMAGE**
FORMULIERUNGEN MIT CERAMIDEN UND/ODER PSEUDOCERAMIDEN UND (ALPHA-)BISABOLOL ZUR BEHANDLUNG VON HAUTSCHÄDEN
FORMULES COMPRENANT DES CÉRAMIDES ET/OU DES PSEUDOCÉRAMIDES ET DE L`ALPHA-BISABOLOL ET APPLICATION À LA RÉPARATION DE PEAU ENDOMMAGÉE

(30) Priority: 22.11.2004 US 630433 P
(43) Date of publication of application: 15.08.2007
(73) Proprietor: Symrise GmbH & Co. KG, 37603 Holzminden (DE)
(72) Inventor: VIELHABER, Gabriele, 37603 Holzminden (DE)
(74) Representative: Stilkenböhmer, Uwe Michael
(86) International application number: PCT/EP2005/056156
(87) International publication number: WO 2006/053912

(56) References cited:
- EP-B- 0 785 952
- WO-A-03/075862
- US-A- 5 391 373
- US-A- 5 882 665
- US-A1- 2004 076 660
- US-B1- 6 335 388
- US-B1- 6 495 123

## Description

The present invention relates to formulations and uses for strengthening the barrier function of damaged and undamaged skin. Within the framework of the invention, mixtures are used which contain (a) one or more pseudoceramides or ceramides and pseudoceramides, and (b) (alpha-)bisabolol (bisabolol, preferably alpha-bisabolol) according to claim 1. The mixtures preferably also comprise (c) cholesterol or phytosterols, and (d) one or more fatty acids.

The skin is the largest organ of the body. It protects the body from uncontrolled water loss and environmentally induced mechanical, physical, biological and chemical stress. This protective function is fulfilled primarily by the so-called epidermal permeability barrier. It is located in the uppermost skin layer, the stratum corneum epidermidis, and consists of a compact composite of multiple intercellular lipid lamellae and corneal cells embedded therein.

The gums and oral mucosa are also equipped with a permeability barrier whose essential constituent consists of intercellular lipid lamellae [Law et al. (1995) Arch. Oral Biol. 40, 1085-1091]. Finally, hair is also protected from environmental influences by embedded lipids [Wix et al. (1987) Comp. Biochem. Physiol. B. 86, 671-673].

Numerous investigations prove that the intercellular lipid lamellae are composed of cholesterol, ceramides and fatty acids in a molar ratio of 1:1:1. The ceramide class of lipids is of particular importance here because on the one hand ceramides make up almost 50% of the proportion by weight of the barrier lipids. On the other hand, special ceramides carrying a long-chain omega-hydroxy fatty acid (C30-32) facilitate covalent bonding to glutamate residues of surface proteins of the corneal cells. This ensures that the permeability barrier has a particularly rigid structure.

A number of factors are known which damage the barrier of the skin (scalp) and hair, including excessive treatment with detergents or solvents, irradiation with UV light, or excessively low atmospheric humidity. However, a disturbance of the barrier also characterizes numerous eczematous syndromes such as atopic and seborrhoeic dermatitis and dandruff, or severe hereditary diseases such as Gaucher's disease. Finally, the barrier function of the skin is observed to deteriorate with advancing age.

A reduced or disturbed barrier function is accompanied by increased amounts of inflammatory mediators, especially interleukin-1 (Wood et al., J. Clin. Invest. 1992, 90, 482-7; Altemus et al., J. Invest. Dermatol. 2001, 117, 309-17), causing irritant skin reactions.

If the barrier function is reduced, topically applied substances such as allergens and irritants can penetrate more easily into the skin, where they can develop more severe effects than in undamaged skin. Attempts have therefore already been made to strengthen the barrier function of the skin in order to prevent allergic and irritant skin reactions or repair the skin. Corresponding active substances have been described in DE4420625 and DE3330628 (bisabolol and/or panthenol), W002069911, DE10111045, WO9712598, WO01021150 (stimulators of ceramide biosynthesis) and FR2811228 (oligosaccharides in combination with ceramide 2).

Another possible way of preventing or repairing said skin damage is the topical application of dermatological compositions containing ceramide and pseudoceramide [Kucharekova et al. (2002) Contact Dermatitis 46, 331-338; Coderch et al. (2002) Contact Dermatitis 47, 139-146; Park et al. (2001) Cosmetics & Toiletries 116(6), 65-76; Park et al. (2001) SÖFW-Journal 127, 10-18].

Natural ceramides are difficult to isolate and they are also laborious and expensive to synthesize. Moreover, it is difficult to process natural ceramides in emulsions because of their high melting point, so structural analogues of ceramides (pseudoceramides) have been developed.

Ceramides and pseudoceramides are amphiphilic molecules consisting of a polar head group and a non-polar "tail" made up of two optionally hydroxyl-substituted long (≥C6) alkyl or alkenyl chains; cf. the structure of ceramide 2 shown below:

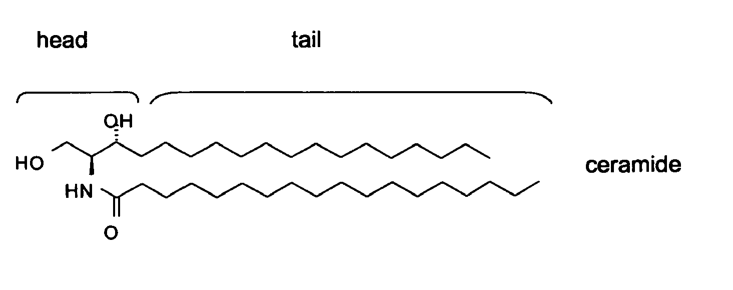

Amphiphilic compounds of this form can be incorporated particularly well into the double membrane of the skin's lipid barrier.

US06060612 describes the synthesis of 1,3-bis(N-(2-hydroxyalkyl)acylamino)-2-hydroxyalkanes as pseudoceramides, and their cosmetic use.

WO9821176 describes the preparation of N-(2-hydroxyethyl)-3-oxo-2-alkylalkylamides and their cosmetic use for protecting against skin ageing and for strengthening the resistance of skin and hair and repairing them.

EP 0864563 A1 describes the use of N-acylhydroxyamino acid esters, especially N-acylhydroxyproline and -threonine esters, for strengthening the natural barrier function in order to protect against external influences and irritations.

Other examples of pseudoceramides are given in Möller H., 2002 [The Chemistry of Natural and Synthetic Skin Barrier Lipids. In: Jungermann E., Cosmetic Science and Technology, vol. 24, Cosmetic Lipids and the Skin Barrier. Marcel Dekker New York Basel 2002, 1-35] and in the CTFA Ingredient Database [http://www.ctfa-online.org/pls/cffa//ctfa_online.home (where they can be found under the trade name "ceramide", compounds bearing the name "ceramide" in the INCI nomenclature being ceramides and the others being pseudoceramides)].

Research Disclosure no. 468117 (May 2003) describes the synergistic combination of N-acylhydroxyamino acid esters with anti-irritants for skin and hair repair. The combination of 0.1 - 0.3% of alpha-bisabolol with 0.5 or 1.0% of N-palmitoylhydroxyproline esters is given as an example.

WO9400127 describes the use of a therapeutically effective mixture of cholesterol, ceramides, an essential fatty acid and a non-essential fatty acid with a C12 to C20 alkyl chain, in a molar ratio of (2-5):(1-3):(1-3):(1.5-3.5), for restoring the epidermal barrier function of damaged skin.

Park et al. (2001) [SÖFW-Journal 127, 10-18] describe that a mixture of the pseudoceramide 1,3-bis(N-(2-hydroxypropyl)palmitoylamino)-2-hydroxypropane with cholesterol, linoleic acid and palmitic acid in a weight ratio of 1:1:1:1, for a total concentration of the mixture of 1%, repairs acetone-damaged skin more rapidly than 1% of the pseudoceramide by itself.

Patent document EP-B-0 785 952 (PIERRE FABRE DERMO-COSMETIQUE) 15 July 1998 (1998-07-15) discloses on page 12 a "osmo actif après soleil" comprising a ceramide and alpha bisabolol.

The object of the present invention was to provide formulations and uses for strengthening the barrier function of damaged and undamaged (primarily human) skin which are more efficient than those according to the state of the art. The preparations and individual active substances to be used in the methods to be provided according to the invention should be cost-effective and efficient.

The object is achieved according to the invention, in respect of the formulation to be provided, by a cosmetic or pharmaceutical formulation that can be applied to the skin or hair, comprising:
(a) one or more pseudoceramides or ceramides andr pseudoceramides, wherein the pseudoceramid used is N-acylhydroxyamino acid ester of formula (I) as described in claim 1 and
(b) bisabolol, preferably alpha-bisabolol, the proportion by weight of (alpha-)bisabolol (bisabolol, preferably alpha-bisabolol) in the formulation being equal to or greater than the total proportion by weight of ceramides and/or pseudoceramides, and the total amount of components (a) and (b) being sufficient to strengthen the barrier of damaged and/or undamaged skin (especially human skin).

The invention is based on the surprising finding that the extended combination of pseudoceramide or ceramides and pseudoceramides, wherein the pseudoceramide used is N- acylhydroxyamino acid ester of formula (I) as described in claim 1 with (alpha-)bisabolol allows a more efficient repair of and/or protection against skin and hair damage, based on synergistic effects, if the (alpha-)bisabolol is present in an amount equal to or greater than that of the ceramide and/or pseudoceramide. As ceramide and pseudoceramide are generally more expensive than (alpha-)bisabolol, a more cost-effective method is additionally provided by the higher proportion of (alpha-)bisabolol.

The bisabolol used in terms of the present invention can be of natural or synthetic origin. In the context of the present text, the term "alpha-bisabolol" covers (+)-alpha-bisabolol, (-)-alpha-bisabolol, (+)-epi-alph6-bisabolol and (-)-epi-alpha-bisabolol, as well as mixtures of two, three or all of said alpha-bisabolol isomers. In particular the term "alpha-bisabolol" covers racemic mixtures of (+/-)-alpha-bisabolol and/or (+/-)-epi-alpha-bisabolol. The bisabolol used is preferably natural (-)-alpha-bisabolol and/or synthetic alpha-bisabolol. Synthetic alpha-bisabolol is obtainable from Symrise under the name "Dragosantol", for example.

It has further been found that the extended combination of (a) (pseudo)ceramide(s) and (b) (alpha-)bisabolol with (c) cholesterol and/or phytosterol(s) and (d) fatty acid(s) allows an even more efficient repair of skin and hair damage, based on synergistic effects. Skin and hair are also preventively protected against damage more efficiently by the synergistic combination of pseudoceramides with alpha-bisabolol, fatty acids and cholesterol or phytosterols. A corresponding formulation according to the invention is therefore particularly preferred, palmitic acid and stearic acid being preferred fatty acids. Incidentally, in the case of the combination of components (a), (b), (c) and (d), the amount of component (b) present does not have to be equal to or greater than that of component (a).

Components (a), (b), (c) and (d) are advantageously used in a weight ratio of (0.5-3):(1-5):(0.5-3):(0.5-3).

One particularly effective form of application is the coapplication of (a) (pseudo)ceramides and (b) (alpha-)bisabolol with (c) cholesterol and/or phytosterols and (d) essential or non-essential fatty acids in weight ratios of 2:4:1:1, 2:2:1:1, 1:2:1:1, 1:1:1:1 and 1:2:2:1, especially of 2:4:1:1 and 1:1:1:1.

The proportion of component (a) in the formulation ranges preferably from 0.01 to 20 wt.%, particularly preferably from 0.01 to 10 wt.% and very particularly preferably from 0.01 to 5 wt.%. The proportion of component (b) in the formulation ranges preferably from 0.001 to 20 wt.%, particularly preferably from 0.01 to 10 wt.% and very particularly preferably from 0.05 to 5 wt.%, the amount of component (b) (bisabolol) always being at least equal to or in excess relative to the total amount of ceramide and/or pseudoceramide (component (a)).

The formulations according to the invention are prepared by conventional methods known per se, e.g. by incorporating one or more of the following components to be used according to the invention: (a) pseudoceramides of formula (I) and (b) (alpha-)bisabolol and optionally (c) cholesterol and/or phytosterols and (d) fatty acids, into a cosmetic or dermatological base formulation of conventional composition. In addition to its effect of strengthening the skin barrier, the finished formulation according to the invention can also be used e.g. for the treatment, care or cleaning of the skin or hair or as a make-up product in decorative cosmetics.

Preferred formulations according to the invention contain a total preferably of 0.01 wt.% to 30 wt.%, particularly preferably of 0.01 to 20 wt.% and very particularly preferably of 0.05 wt.% to 5 wt.%, based on the total weight of the formulation, of the mixture constituents (a) ceramide/pseudoceramide, (b) (alpha-)bisabolol and optionally (c) cholesterol/phytosterols and (d) fatty acids, and can take the form of soap, syndet, liquid washing, shower and bath preparation, emulsion (as solution, dispersion, suspension, cream, lotion or milk, depending on preparative method and ingredients, of the type W/O, O/W or multiple emulsion, PIT emulsion, emulsion foam, microemulsion, nanoemulsion, Pickering emulsion), ointment, paste, gel (including hydrogel, hydrodispersion gel, oleogel), oil, toner, balsam, serum, powder, eau de toilette, eau de Cologne, perfume, wax, stick, roll-on, (pump) spray, aerosol (foaming, non-foaming or after-foaming), foot care product (including keratolytics, deodorant), pre-shave or after-shave (balm, lotion), depilatory product, hair care product, e.g. shampoo (incl. 2-in-1 shampoo), conditioner, hair treatment, hair tonic, hair rinse, hair cream, pomade, perming and fixing product, hair straightening product (defrizzer, relaxer), hair strengthener, styling aid (e.g. gel or wax), bleach, hair dye (e.g. temporary, direct, semipermanent, permanent hair dye), nail care product, e.g. nail varnish and nail varnish remover, deodorant and/or antiperspirant, mouthwash, make-up, make-up remover or decorative cosmetic (e.g. powder, eye shadow, kajal stick, lipstick).

It can be advantageous to provide the formulations according to the invention in encapsulated form, e.g. in gelatin, wax materials, liposomes, cellulose capsules or cyclodextrin capsules.

Other conventional cosmetic auxiliary substances and additives can be present in formulations according to the invention in amounts advantageously of 5 - 99 wt.%, preferably of 10 - 80 wt.%, based on the total weight of the mixture. The formulations can also contain water in an amount of up to 99.99 wt.%, preferably of 5 - 80 wt.%, based on the total weight of the formulation.

The formulations according to the invention can contain cosmetic auxiliary substances and additives such as those conventionally used in cosmetic preparations, e.g. sunscreens, preservatives, bactericides, fungicides, virucides, cooling substances, insect repellents (e.g. DEET, IR 3225, Dragorepel), plant extracts, antiinflammatory substances, wound healing accelerators (e.g. chitin or chitosan and its derivatives), film-forming substances (e.g. polyvinylpyrrolidones or chitosan or its derivatives), customary antioxidants, vitamins (e.g. vitamin C and derivatives, tocopherols and derivatives, vitamin A and derivatives), 2-hydroxycarboxylic acids (e.g. citric acid, malic acid, L-, D-or DL-lactic acid), skin colourants (e.g. walnut extracts or dihydroxyacetone), active ingredients for promoting hair growth (e.g. minoxidil, diphencyprone, hormones, finasteride, phytosterols such as beta-sitosterol, biotin, or extracts of Cimicifuga racemosa, Eugenia caryophyllata or Hibiscus rosasinensis, barley, hops, or rice or wheat hydrolysates), skin care products (e.g. cholesterol, ceramides, pseudoceramides), softening, moisturizing and/or moisture-retaining substances (e.g. glycerol or urea), fats, oils, saturated fatty acids, monounsaturated or polyunsaturated fatty acids, α-hydroxy acids, polyhydroxy fatty acids or their derivatives (e.g. linoleic acid, α-linolenic acid, γ-linolenic acid or arachidonic acid and their respective natural or synthetic esters), waxes or other conventional constituents of a cosmetic or dermatological formulation, such as alcohols, polyols, polymers, foam stabilizers, electrolytes, organic solvents, silicone derivatives or chelating agents (e.g. ethylenediaminetetraacetic acid and derivatives), antidandruff substances (e.g. climbazole, ketoconazole, piroctonoleamine, zinc pyrithione), hair care products, perfumes, antifoams, dyestuffs, pigments with a colouring action, thickeners (advantageously silicon dioxide, aluminium silicates such as bentonites, polysaccharides or their derivatives, e.g. hyaluronic acid, guar kernel flour, xanthan gum, hydroxypropyl methyl cellulose or allulose derivatives, particularly advantageously polyacrylates such as carbopols, or polyurethanes), surface-active substances, emulsifiers, plant parts and plant extracts (e.g. arnica, aloe, beard lichen, ivy, stinging nettle, ginseng, henna, camomile, marigold, rosemary, sage, horsetail or thyme), animal extracts, e.g. royal jelly or propolis, proteins, protein hydrolysates, yeast extracts, hop and wheat extracts, peptides or thymus extracts.

The amounts of cosmetic or dermatological auxiliary substances and additives and perfume to be used can readily be determined by those skilled in the art on a simple trial-and-error basis, as a function of the particular type of product.

Advantageously the formulations according to the invention contain at least one UVA filter and/or at least one UVB filter and/or at least one inorganic pigment. The mixtures can take a variety of forms, e.g. those conventionally used for sunscreen preparations for protecting the skin and hair from ultraviolet radiation. They can thus form e.g. a solution, an emulsion of the water-in-oil (W/O) type or oil-in-water (O/W) type, or a multiple emulsion, for example of the water-in-oil-in-water (W/O/W) type, a gel, a hydrodispersion, a solid stick or else an aerosol. The total amount of filter substances is from 0.01 wt.% to 40 wt.%, preferably from 0.1% to 10 wt.% and particularly preferably from 1.0 to 5.0 wt.%, based on the total weight of the mixture, in order to provide cosmetic mixtures (preparations).

Examples of advantageous UV filters are:
• p-aminobenzoic acid
• ethyl p-aminobenzoate, ethoxylated (25 mol)
• 2-ethylhexyl p-dimethylaminobenzoate
• ethyl p-aminobenzoate, N-propoxylated (2 mol)
• glyceryl p-aminobenzoate
• homomenthyl salicylate (homosalate) (Neo Heliopan^{®}HMS)
• 2-ethylhexyl salicylate (Neo Heliopan^{®}OS)
• triethanolamine salicylate
• 4-isopropylbenzyl salicylate
• menthyl anthranilate (Neo Heliopan^{®}MA)
• ethyl diisopropylcinnamate
• 2-ethylhexyl p-methoxycinnamate (Neo Heliopan^{®}AV)
• methyl diisopropylcinnamate
• isoamyl p-methoxycinnamate (Neo Heliopan^{®}E 1000)
• p-methoxycinnamic acid diethanolamine salt
• isopropyl p-methoxycinnamate
• 2-ethylhexyl 2-cyano-3,3-diphenylacrylate (Neo Heliopan^{®}303)
• ethyl 2-cyano-3,3'-diphenylacrylate
• 2-phenylbenzimidazolesulfonic acid and salts (Neo Heliopan^{®}Hydro)
• 3-(4'-trimethylammonium)benzylidenebornan-2-one methylsulfate
• terephthalylidenedibornanesulfonic acid and salts (Mexoryl^{®}SX)
• 4-t-butyl-4'-methoxydibenzoylmethane (avobenzone)/(Neo Heliopan^{®}357)
• β-imidazol-4(5)-acrylic acid (urocanic acid)
• 2-hydroxy-4-methoxybenzophenone (Neo Heliopan^{®}BB)
• 2-hydroxy-4-methoxybenzophenone-5-sulfonic acid
• dihydroxy-4-methoxybenzophenone
• 2,4-dihydroxybenzophenone
• tetrahydroxybenzophenone
• 2,2'-dihydroxy-4,4'-dimethoxybenzophenone
• 2-hydroxy-4-n-octyloxybenzophenone
• 2-hydroxy-4-methoxy-4'-methylbenzophenone
• 3-(4'-sulfo)benzylidenebornan-2-one and salts
• 3-(4'-methylbenzylidene)-d,l-camphor (Neo Heliopan^{®}MBC)
• 3-benzylidene-d,l-camphor
• 4-isopropyldibenzoylmethane
• 2,4,6-trianilino(p-carbo-2'-ethylhexyl-1'-oxy)-1,3,5-triazine
• phenylenebisbenzimidazyltetrasulfonic acid disodium salt (Neo Heliopan^{®}AP)
• 2,2'-(1,4-phenylene)bis(1H-benzimidazole-4,6-disulfonic acid) monosodium salt
• N-[(2 and 4)-[2-(oxoborn-3-ylidene)methyl]benzyl]acrylamide polymer
• phenol,2-(2H-benzotriazol-2-yl)-4-methyl-6-(2-methyl-3-(1,3,3,3-tetramethyl-1-((trimethylsilyl)oxy)disiloxanyl)propyl) (Mexoryl^{®}XL)
• 4,4'-[(6-[4-(1,1-dimethyl)aminocarbonyl)phenylamino]-1,3,5-triazine-2,4-diyldiimino]bis(benzoic acid 2-ethylhexyl ester) (Uvasorb^{®}HEB)
• 2,2'-methylenebis(6-(2H-benztriazol-2-yl)-4-(1,1,3,3-tetramethylbutyl)phenol) (Tinosorb^{®}M)
• 2,4-bis[4-(2-ethylhexyloxy)-2-hydroxyphenyl]-1,3,5-triazine
• benzylidene malonate polysiloxane (Parsol^{®}SLX)
• glyceryl ethylhexanoate dimethoxycinnamate
• disodium 2,2'-dihydroxy-4,4'-dimethoxy-5,5'-disulfobenzophenone
• dipropylene glycol salicylate
• sodium hydroxymethoxybenzophenonesulfonate
• 4,4',4-(1,3,5-triazine-2,4,6-triyltriimino)tris(benzoic acid 2-ethylhexyl ester) (Uvinul^{®}T150)
• 2,4-bis[{4-(2-ethylhexyloxy)-2-hydroxy}phenyl]-6-(4-methoxyphenyl)-1,3,5-triazine (Tinosorb^{®}S)
• 2,4-bis[{(4-(3-sulfonato)-2-hydroxypropoxy)-2-hydroxy}phenyl]-6-(4-methoxyphenyl)-1,3,5-triazine sodium salt
• 2,4-bis[{(3-(2-propoxy)-2-hydroxypropoxy)-2-hydroxy}phenyl]-6-(4-methoxyphenyl)-1,3,5-triazine
• 2,4-bis[{4-(2-ethylhexyloxyl)-2-hydroxy}phenyl]-6-[4-(2-methoxyethylcarbonyl)phenylamino]-1,3,5-triazine
• 2,4-bis[{4-(3-(2-propoxy)-2-hydroxypropoxy)-2-hydroxy}phenyl]-6-[4-(2-ethylcarboxy)phenylamino]-1,3,5-triazine
• 2,4-bis[{4-(2-ethylhexyloxy)-2-hydroxy}phenyl]-6-(1-methylpyrrol-2-yl)-1,3,5-triazine
• 2,4-bis[{4-tris(trimethylsiloxysilylpropoxy)-2-hydroxy}phenyl]-6-(4-methoxyphenyl)-1,3,5-triazine
• 2,4-bis[{4-(2"-methylpropenyloxy)-2-hydroxy}phenyl]-6-(4-methoxyphenyl)-1,3,5-triazine
• 2,4-bis[{4-(1',1',1',3',5',5',5'-heptamethylsiloxy-2"-methylpropoxy)-2-hydroxy}phenyl]-6-(4-methoxyphenyl)-1,3,5-triazine
• hexyl 2-(4-diethylamino-2-hydroxybenzoyl)benzoate (Uvinul^{®} A Plus)
• indanylidene compounds according to DE 100 55 940 (= WO 02/38537)

The following UV absorbers are particularly suitable for combination:
• p-aminobenzoic acid
• 3-(4'-trimethylammonium)benzylidenebornan-2-one methylsulfate
• homomenthyl salicylate (Neo Heliopan^{®}HMS)
• 2-hydroxy-4-methoxybenzophenone (Neo Heliopan^{®}BB)
• 2-phenylbenzimidazolesulfonic acid (Neo Heliopan^{®}Hydro)
• terephthalylidenedibornanesulfonic acid and salts (Mexoryl^{®}SX)
• 4-tert-butyl-4'-methoxydibenzoylmethane (Neo Heliopan^{®}357)
• 3-(4'-sulfo)benzylidenebornan-2-one and salts
• 2-ethylhexyl 2-cyano-3,3-diphenylacrylate (Neo Heliopan^{®}303)
• N-[(2 and 4)-[2-(oxobom-3-ylidene)methyl]benzyl]acrylamide polymer
• 2-ethylhexyl p-methoxycinnamate (Neo Heliopan^{®}AV)
• ethyl p-aminobenzoate, ethoxylated (25 mol)
• isoamyl p-methoxycinnamate (Neo Heliopan^{®}E1000)
• 2,4,6-trianilino(p-carbo-2'-ethylhexyl-1'-oxy)-1,3,5-triazine (Uvinul^{®}T150)
• pheno),2-(2H-benzotriazol-2-yl)-4-methyl-6-(2-methy)-3-(1,3,3,3-tetramethyl-1-((trimethylsilyl)oxy)disiloxanyl)-propyl) (Mexoryl^{®}XL)
• 4,4'-[(6-[4-(1,1-dimethyl)aminocarbonyl)phenylamino]-1,3,5-triazine-2,4-diyldiimino]bis(benzoic acid 2-ethylhexyl ester) (Uvasorb^{®}HEB)
• 3-(4'-methylbenzylidene)-d,l-camphor (Neo Helipan^{®}MBC)
• 3-benzylidenecamphor
• 2-ethylhexyl salicylate (Neo Helipan^{®}OS)
• 2-ethylhexyl 4-dimethylaminobenzoate (Padimate O)
• hydroxy-4-methoxybenzophenone-5-sulfonic acid and Na salt
• 2,2'-methylenebis(6-(2H-benztriazol-2-yl)-4-(1,1,3,3-tetramethylbutyl)phenol) (Tinosorb^{®}M)
• phenylenebisbenzimidazyltetrasulfonic acid disodium salt (Neo Heliopan^{®}AP)
• 2,4-bis[{4-(2-ethylhexyloxy)-2-hydroxy}phenyl]-6-(4-methoxyphenyl)-1,3,5-triazine (Tinosorb^{®}S)
• benzylidene malonate polysiloxane (Parsol^{®}SLX)
• menthyl anthranilate (Neo Heliopan^{®}MA)
• hexyl 2-(4-diethylamino-2-hydroxybenzoyl)benzoate (Uvinul^{®} A Plus)
• indanylidene compounds according to DE 100 55 940 (= WO 02/38537)

Advantageous inorganic light-protecting pigments are finely disperse metal oxides and metal salts, for example titanium dioxides, zinc oxide (ZnO), iron oxides (e.g. Fe₂O₃), aluminium oxide (Al₂O₃), cerium oxides (e.g. Ce₂O₃), manganese oxides (e.g. MnO), zirconium oxide (ZrO₂), silicon oxide (SiO₂), mixed oxides of the corresponding metals, and mixtures of such oxides, barium sulfate and zinc stearate. Particularly preferred pigments are those based on TiO₂ or zinc oxide. In preferred embodiments the particles have a mean diameter of less than 100 nm, preferably of between 5 and 50 nm and particularly preferably of between 15 and 30 nm. They can have a spherical shape, but it is also possible to use particles with an ellipsoid shape or a shape that differs from spherical in some other way. The pigments can also be surface-treated, i.e. hydrophilized or hydrophobized. Typical examples are coated titanium dioxides, e.g. titanium dioxide T 805 (Degussa) or Eusolex^{®} T2000 (Merck), or coated zinc oxide, e.g. zinc oxide NDM, suitable hydrophobic coating agents being primarily silicones and especially trialkoxyoctylsilanes or simethicones. So-called micropigments or nanopigments are preferably used in sunscreen products, zinc micropigments or nanopigments being particularly preferred.

The total amount of inorganic pigments, especially hydrophobic inorganic micropigments, in the finished cosmetic or dermatological formulations advantageously ranges from 0.1 to 30 wt.%, preferably from 0.1 to 10.0 and
particularly preferably from 0.5 to 6.0 wt.%, based on the total weight of the formulations.

Other anti-irritants apart from (alpha-)bisabolol can also be used in formulations according to the invention. Anti-irritants can be any anti-inflammatory or redness-alleviating and antipruritic substances that are suitable or customary for cosmetic and/or dermatological applications. Preferred anti-inflammatory or redness-alleviating and antipruritic substances (anti-irritants) are steroidal anti-inflammatory substances of the corticosteroid type, e.g. hydrocortisone, dexamethasone, dexamethasone phosphate, methylprednisolone or cortisone, it being possible to extend the list by adding other steroidal anti-inflammatories. Non-steroidal anti-inflammatories can also be used. The following may be mentioned as examples: oxicams such as piroxicam or tenoxicam; salicylates such as aspirin, Disalcid, Solprin or fendosal; acetic acid derivatives such as diclofenac, fendofenac, indomethacin, sulindac, tolmetin or clindanac; fenamates such as mefenamic, meclofenamic, flufenamic or niflumic; propionic acid derivatives such as ibuprofen, naproxen or benoxaprofen; or pyrazoles such as phenylbutazone, oxyphenylbutazone, febrazone or azapropazone. Natural anti-inflammatory or redness-alleviating and antipruritic substances can be used as alternatives, possibilities being plant extracts, special potent plant extract fractions, and high-purity active substances isolated from plant extracts. Particular preference is given to extracts, fractions and active substances from camomile, Aloe vera, Commiphora species, Rubia species, Echinacea species, willows, willow herb, oats, black and green tea, gingko, coffee, pepper, redcurrant/blackcurrant, tomato, vanilla and almonds, as well as pure substances such as, inter alia, apigenin-7-glucoside, boswellic acid, phytosterols, glycyrrhizinic acid, glabridin or licochalcone A.

In terms of the invention, particular preference is given to panthenol, boswellic acid and extracts and isolated high-purity active substances from oats (e.g. avenanthramides) and Echinacea, and mixtures thereof.

The formulations according to the invention can also contain antioxidants, it being possible to use any antioxidants suitable or customary for cosmetic and/or dermatological applications. The antioxidants are advantageously selected from the group comprising amino acids (e.g. glycine, histidine, tyrosine, tryptophan) and derivatives thereof, imidazoles (e.g. urocanic acid) and derivatives thereof, peptides such as D,L-carnosine, D-camosine, L-carnosine and derivatives thereof (e.g. anserine), carotenoids, carotenes (e.g. α-carotene, β-carotene, lycopene) and derivatives thereof, chlorogenic acid and derivatives thereof, lipoic acid and derivatives thereof (e.g. dihydrolipoic acid), aurothioglucose, propylthiouracil and other thiols (e.g. thioredoxin, glutathione, cysteine, cystine, cystamine and their glycosyl, N-acetyl, methyl, ethyl, propyl, amyl, butyl, lauryl, palmitoyl, oleyl, γ-linoleyl, cholesteryl and glyceryl esters) and their salts, dilauryl thiodipropionate, distearyl thiodipropionate, thiodipropionic acid and derivatives thereof (esters, ethers, peptides, lipids, nucleotides, nucleosides and salts), sulfoximine compounds (e.g. buthionine sulfoximine, homocysteine sulfoximine, buthionine sulfone, penta-, hexa-, heptathionine sulfoximine) in very small tolerable doses, (metal) chelators, e.g. α-hydroxy fatty acids, palmitic acid, phytic acid, lactoferrin, α-hydroxy acids (e.g. citric acid, lactic acid, malic acid), humic acid, bile acid, bile extracts, bilirubin, biliverdin, EDTA, EGTA and derivatives thereof, unsaturated fatty acids and derivatives thereof (e.g. γ-linolenic acid, linoleic acid, oleic acid), folic acid and derivatives thereof, ubiquinone and ubiquinol and derivatives thereof, vitamin C and derivatives (e.g. ascorbyl palmitate, Mg ascorbylphosphate, ascorbyl acetate, ascorbyl glycosides such as 6-O-acyl-2-O-α-D-glucopyranosyl-L-ascorbic acid, 6-O-acyl-2-O-β-D-glucopyranosyl-L-ascorbic acid, 2-O-α-D-glucopyranosyl-L-ascorbic acid or 2-O-β-D-glucopyranosyl-L-ascorbic acid), tocopherols and derivatives thereof (e.g. vitamin E acetate), vitamin A and derivatives thereof (vitamin A palmitate), coniferyl benzoate from benzoin, rutic acid and derivatives thereof, alpha-glycosylrutin, quercetin and derivatives thereof, rosmaric acid, carnosol, carnosolic acid, resveratrol, caffeic acid and derivatives thereof, sinapic acid and derivatives thereof, ferulic acid and derivatives thereof, furfurylideneglucitol, curcuminoids, butylhydroxytoluene, butylhydroxyanisole, nordihydroguaiacic acid, nordihydroguaiaretic acid, trihydroxybutyrophenone, uric acid and derivatives thereof, mannose and derivatives thereof, superoxide dismutase, zinc and derivatives thereof (e.g. ZnO, ZnSO₄), selenium and derivatives thereof (e.g. selenium methionine), stilbenes and derivatives thereof (e.g. stilbene oxide, trans-stilbene oxide), derivatives (salts, esters, ethers, sugars, nucleotides, nucleosides, peptides and lipids) of said active substances, or antioxidative extracts or fractions of plants such as green tea, rooibos, honeybush, grape, rosemary, sage, balm, thyme, lavender, olive, oats, cacao, gingko, ginseng, liquorice, honeysuckle, Sophora, Pueraria, Pinus, Citrus, Phyllanthus emblica or St John's wort.

The amount of antioxidants (one or more compounds) in the formulations according to the invention is preferably 0.01 to 20 wt.%, particularly preferably 0.05 - 10 wt.% and very particularly preferably 0.2 - 5 wt.%, based on the total weight of the preparation.

If vitamin E and/or its derivatives represent the antioxidant(s), their respective concentrations are advantageously chosen from the range between 0.001 and 10 wt.%, based on the total weight of the formulation.

If vitamin A or vitamin A derivatives, or carotenes or their derivatives, represent the antioxidant(s), their respective concentrations are advantageously chosen from the range between 0.001 and 10 wt.%, based on the total weight of the formulation.

The (cosmetic) formulations according to the invention can also contain active substances and active substance combinations for combating skin ageing and wrinkling. It is possible here, according to the invention, to use any active substances for combating skin ageing and wrinkling that are suitable or customary for cosmetic and/or dermatological applications. In this respect, advantageous active substances for combating skin ageing and wrinkling are soya protein or protein hydrolysates, soya isoflavones, hydrolysed rice protein, hydrolysed hazelnut protein, oligopeptides from hydrolysed Hibiscus esculentus extract, wheat protein, β-glucans, e.g. from oats, and derivatives thereof, glycoproteins, ursolic acid and its salts, betulin, betulinic acid and its salts, retinol, retinol palmitate, propyl gallate, precocenene, 6-hydroxy-7-methoxy-2,2-dimethyl-1 (2H)-benzopyran, 3,4-dihydro-6-hydroxy-7-methoxy-2,2-dimethyl-1(2H)-benzopyran, creatine, or other synthetic or natural active substances for combating skin ageing and wrinkling, it also being possible for the latter to be used in the form of an extract of plants such as green tea, Rubus fruticosus, Sanguisorba officinalis, Centella asiatica, Ribes nigrum, Passiflora incarnata, Phyllanthus emblica, okra, algae, evening primrose, rosemary, sage, Echinacea, birch, apple or soya.

β-Glucan is particularly preferably used as another active substance for combating skin ageing; 1,3-1,4-linked β-glucan from oats, Rubus fruticosus extract or wheat protein is very particularly preferred.

Formulations according to the invention in the form of a cosmetic preparation can advantageously also contain moisturizers. The following substances are examples of moisturizers used: sodium lactate, urea and urea derivatives, alcohols, glycerol, diols such as propylene glycol, 1,2-pentanediol, 1,2-hexanediol and 1,2-octanediol, collagen, elastin or hyaluronic acid, diacyl adipates, petrolatum, urocanic acid, lecithin, panthenol, phytantriol, lycopene, (pseudo)ceramides, glycosphingolipids, cholesterol, phytosterols, chitosan, chondroitin sulfate, lanolin, lanolin esters, amino acids, alpha-hydroxy acids (e.g. citric acid, lactic acid, malic acid) and derivatives thereof, mono-, di- and oligosaccharides such as glucose, galactose, fructose, mannose, fruit sugar and lactose, polysugars such as β-glucans, especially 1,3-1,4-β-glucan from oats, alpha-hydroxy fatty acids, triterpene acids such as betulinic acid or ursolic acid, and algae extracts.

The formulations according to the invention can also be used together with osmolytes. The following may be mentioned as examples of osmolytes: substances from the group comprising sugar alcohols (myoinositol, mannitol, sorbitol), quaternary amines such as taurine, choline, betaine, betaine glycine and ectoine, diglyceryl phosphate, phosphorylcholine, glycerophosphorylcholine, amino acids such as glutamine, glycine, alanine, glutamate, aspartate or proline, phosphatidylcholine, phosphatidylinositol, inorganic phosphates, and polymers of said compounds such as proteins, peptides, polyamino acids and polyols. All osmolytes have a skin-moisturizing effect at the same time.

Preferably, formulations according to the invention can also contain active substances which stimulate skin and hair tinting or bronzing in a chemical or natural way, thereby achieving a more rapid action based on synergistic effects. Particularly preferably, said substances are substrates or substrate analogues of tyrosinase, such as L-tyrosine, L-DOPA or L-dihydroxyphenylalanine, stimulators of tyrosinase activity or expression, such as theophylline, caffeine, proopiomelanocortin peptides such as ACTH, alpha-MSH, their peptide analogues and other substances that bind to the melanocortin receptor, peptides such as Val-Gly-Val-Ala-Pro-Gly, Lys-Ile-Gly-Arg-Lys or Leu-Ile-Gly-Lys, purines, pyrimidines, folic acid, copper salts such as copper gluconate, chloride or pyrrolidonate, flavonoids, flavanone glycosides such as naringin and hesperidin, 1,3,4-oxadiazole-2-thiols such as 5-pyrazin-2-yl-1,3,4-oxadiazole-2-thiol, melanin derivatives such as Melasyn-100 and MelanZe, diacylglycerols, aliphatic or cyclic diols, psoralenes, prostaglandins and their analogues, adenylate cyclase activators, and compounds which activate the transfer of melanosomes into keratinocytes, such as serine proteases or PAR-2 receptor agonists, extracts of plants and plant parts of Chrysanthemum species and Sanguisorba species, walnut extracts, urucum extracts, rhubarb extracts, erytrulose and dihydroxyacetone.

The formulations according to the invention can advantageously be used in numerous cases in combination with skin-lightening substances. Any skin-lightening substances that are conventional or customary for cosmetic and/or dermatological applications can be used according to the invention. Advantageous skin-lightening substances in this respect are koji acid (5-hydroxy-2-hydroxymethyl-4-pyranone), koji acid derivatives, e.g. koji acid dipalmitate, arbutin, ascorbic acid, ascorbic acid derivatives, hydroquinone, hydroquinone derivatives, resorcinol, sulfur-containing molecules, e.g. glutathione or cysteine, alpha-hydroxy acids (e.g. citric acid, lactic acid, malic acid) and derivatives thereof, N-acetyltyrosine and derivatives, undecenoylphenylalanine, gluconic acid, 4-alkylresorcinols, chromone derivatives such as aloesin, flavonoids, thymol derivatives, 1-aminoethylphosphinic acid, thiourea derivatives, ellagic acid, nicotinamide, zinc salts such as zinc chloride or gluconate, thujaplicin and derivatives, triterpenes such as maslinic acid, sterols such as ergosterol, benzofuranones such as senkyunolide, vinyl-and ethylguaiacol, inhibitors of nitrogen oxide synthesis, e.g. L-nitroarginine and derivatives thereof, 2,7-dinitroindazole or thiocitrulline, metal chelators (e.g. α-hydroxy fatty acids, palmitic acid, phytic acid, lactoferrin, humic acid, bile acid, bile extracts, bilirubin, biliverdin, EDTA, EGTA and derivatives thereof), retinoids, soya milk, serine protease inhibitors, lipoic acid or other synthetic or natural active substances for lightening the skin and hair, the latter also being used in the form of plant extracts, e.g. bearberry extract, rice extract, liquorice root extract or constituents obtained therefrom by enrichment, such as glabridin or licochalcone A, Artocarpus extract, extract of Rumex and Ramulus species, extracts of pine species (Pinus) and extracts of Vitis species or stilbene derivatives obtained therefrom by enrichment, and extracts of Saxifraga, mulberry, Scutelleria and/or grapes.

Formulations according to the invention in the form of cosmetic preparations can also contain anionic, cationic, non-ionic and/or amphoteric surfactants, especially if crystalline or microcrystalline solids, for example inorganic micropigments, are to be incorporated into the mixtures.

Anionic surfactants normally have carboxylate, sulfate or sulfonate groups as functional groups. In aqueous solution they form negatively charged organic ions in an acidic or neutral medium. Cationic surfactants are almost exclusively characterized by the presence of a quaternary ammonium group. In aqueous solution they form positively charged organic ions in an acidic or neutral medium. Amphoteric surfactants contain both anionic and cationic groups and accordingly behave as anionic or cationic surfactants in aqueous solution, depending on the pH. They have a positive charge in a strongly acidic medium and a negative charge in an alkaline medium. In the neutral pH range, on the other hand, they are zwitterionic. Non-ionic surfactants typically have polyether chains and do not form ions in an aqueous medium.

### A. Anionic surfactants

Anionic surfactants that can advantageously be used are acylamino acids (and their salts) such as:
- acylglutamates, for example sodium acylglutamate, di-TEA palmitoylaspartate and sodium caprylic/capric glutamate,
- acylpeptides, for example palmitoyl-hydrolysed milk protein, sodium cocoyl-hydrolysed soya protein and sodium/ potassium cocoyl-hydrolysed collagen,
- sarcosinates, for example myristoyl sarcosine, TEA lauroylsarcosinate, sodium lauroylsarcosinate and sodium cocoylsarcosinate,
- taurates, for example sodium lauroyltaurate and sodium methylcocoyltaurate,
- acyllactylates, lauroyllactylate, caproyllactylate, stearoyllactylate,
- alaninates,
   carboxylic acids and derivatives, such as:
   lauric acid, aluminium stearate, magnesium alkanolate and zinc undecylenate,

- carboxylic acid esters, for example calcium stearoyllactylate, laureth-6 citrate and sodium PEG-4 lauramidecarboxylate,
- carboxylic acid ethers, for example sodium laureth-13 carboxylate and sodium PEG-6 cocamidecarboxylate,
- phosphoric acid esters and salts, such as DEA oleth-10 phosphate and dilaureth-4 phosphate,
   sulfonic acids and salts, such as:
- acylisethionates, e.g. sodium/ammonium cocoylisethionate,
- alkylarylsulfonates,
- alkylsulfonates, for example sodium cocomonoglyceridesulfate, sodium C₁₂₋₁₄-olefinsulfonate, sodium laurylsulfoacetate and magnesium PEG-3 cocamidesulfate,
- sulfosuccinates, for example sodium dioctylsulfosuccinate, disodium laureth sulfosuccinate, disodium laurylsulfosuccinate and disodium undecylenamido MEA sulfosuccinate,
   and
   sulfuric acid esters such as:
- alkyl ether sulfate, for example sodium, ammonium, magnesium, MIPA and TIPA laureth sulfate, sodium myreth sulfate and sodium C12-13 pareth sulfate,
- alkylsulfates, for example sodium, ammonium and TEA laurylsulfate.

### B. Cationic surfactants

The following cationic surfactants can advantageously be used:
- alkylamines,
- alkylimidazoles,
- ethoxylated amines and
- quaternary surfactants:

   RNH₂CH₂CH₂COO⁻ (at pH = 7)

   RNHCH₂CH₂COO- B⁺ (at pH = 12), B⁺ = any cation, e.g. Na⁺
- esterquats

Quaternary surfactants contain at least one N atom covalently bonded to 4 alkyl or aryl groups. This results in a positive charge, independently of the pH. Alkylbetaine, alkylamidopropylbetaine and alkylamidopropylhydroxysulfaine are advantageous. The cationic surfactants used can also preferably be selected from the group comprising quaternary ammonium compounds, especially benzyltrialkylammonium chlorides or bromides, for example benzyldimethylstearylammonium chloride, alkyltrialkylammonium salts, for example cetyltrimethylammonium chloride or bromide, alkyldimethylhydroxyethylammonium chlorides or bromides, dialkyldimethylammonium chlorides or bromides, alkylamidoethyltrimethylammonium ether sulfates, alkylpyrimidinium salts, for example laurylpyrimidinium or cetylpyridinium chloride, imidazoline derivatives, and compounds of cationic character, such as amine oxides, for example alkyldimethylamine oxides or alkylaminoethyldimethylamine oxides. Cetyltrimethylammonium salts can be used to particular advantage.

### C. Amphoteric surfactants

The following amphoteric surfactants can advantageously be used:
- acyl/dialkylethylenediamine, for example sodium acylamphoacetate, disodium acylamphodipropionate, disodium alkylamphodiacetate, sodium acylamphohydroxypropylsulfonate, disodium acylamphodiacetate and sodium acylamphopropionate,
- N-alkylamino acids, for example aminopropylalkylglutamide, alkylaminopropionic acid, sodium alkylimidodipropionate and lauroamphocarboxyglycinate.

### D. Non-ionic surfactants

The following non-ionic surfactants can advantageously be used:
- alcohols,
- alkanolamides such as cocamides MEA/DEA/MIPA,
- amine oxides such as cocamidopropylamine oxide,
- esters formed by the esterification of carboxylic acids with ethylene oxide, glycerol, sorbitan or other alcohols,
- ethers, for example ethoxylated/propoxylated alcohols,
   ethoxylated/propoxylated esters, ethoxylated/propoxylated glycerol esters, ethoxylated/propoxylated cholesterols, ethoxylated/propoxylated triglyceride esters, ethoxylated/ propoxylated lanolin, ethoxylated/propoxylated polysiloxanes, propoxylated POE ethers, and alkylpolyglycosides such as laurylglucoside, decylglycoside and cocoglycoside,
- sucrose esters and ethers,
- polyglycerol esters, diglycerol esters and monoglycerol esters,
- methylglucose esters, hydroxy acid esters.

It is also advantageous to use a combination of anionic and/or amphoteric surfactants with one or more non-ionic surfactants.

The surface-active substance can be present in a concentration of between 1 and 98 wt.% in the mixtures to be used according to the invention, based on the total weight of the mixture.

A lipid phase in formulations according to the invention can advantageously be selected from the following groups of substances:
- mineral oils (advantageously paraffin oil), mineral waxes,
- fatty oils, fats, waxes and other natural and synthetic fatty substances, preferably esters of fatty acids with alcohols of low C number, e.g. with isopropanol, propylene glycol or glycerol, or esters of fatty alcohols with alkanoic acids of low C number or with fatty acids,
- alkyl benzoates,
- silicone oils such as dimethylpolysiloxanes, diethylpolysiloxanes, diphenylpolysiloxanes and mixed forms thereof,
- hydrocarbons (advantageously squalane or squalene),
- synthetic or semisynthetic triglyceride oils (e.g. triglycerides of capric or caprylic acid),
- natural oils (one or more nurturing animal and/or vegetable fats and oils, such as olive oil, sunflower oil, refined soya oil, palm oil, sesame oil, rapeseed oil, almond oil, borage oil, evening primrose oil, coconut oil, shea butter, jojoba oil, oat oil, sperm oil, tallow, neatsfoot oil and lard),
and optionally other nurturing constituents, for example fatty alcohols having 8-30 C atoms, it being possible for the latter to be saturated or unsaturated and linear or branched. Examples of fatty alcohols which can be used are decanol, decenol, octanol, octenol, dodecanol, dodecenol, octadienol, decadienol, dodecadienol, oleyl alcohol, ricinoleyl alcohol (9-cis-octadecene-1,12-diol), erucyl alcohol, stearyl alcohol, isostearyl alcohol, cetyl alcohol, lauryl alcohol, myristyl alcohol, arachidyl alcohol, caprylic alcohol, capric alcohol, linoleyl alcohol, linolenyl alcohol and behenyl alcohol, and their Guerbet alcohols, the list being extendable almost without limit by other alcohols of chemically related structure. The fatty alcohols preferably originate from natural fatty acids and are conventionally prepared from the corresponding fatty acid esters by reduction. It is also possible to use fatty alcohol fractions formed by reduction from naturally occurring fats and fatty oils, e.g. tallow, groundnut oil, colza oil, cottonseed oil, soya oil, sunflower oil, palm kernel oil, linseed oil, maize oil, castor oil, rapeseed oil, sesame oil, cacao butter and coconut fat. Synthetic ester oils may also be present. Preferred esters are those of saturated and/or unsaturated, linear and/or branched alkanecarboxylic acids having 3 to 30 C atoms with saturated and/or unsaturated, linear and/or branched alcohols having 3 to 30 C atoms, and esters of aromatic carboxylic acids with saturated and/or unsaturated, linear and/or branched alcohols having 3 to 30 C atoms, selected especially from the group comprising isopropyl myristate, isopropyl stearate, isopropyl palmitate, isopropyl oleate, n-butyl stearate, n-hexyl laurate, n-decyl laurate, isooctyl stearate, isononyl stearate, isononyl isononanoate, 2-ethylhexyl palmitate, 2-ethylhexyl laurate, 2-ethylhexyl ethylhexanoate, cetearyl 2-ethylhexanoate, 3,5,5-trimethylhexyl 3,5,5-trimethylhexanoate, 2-ethylhexyl isononanoate, 2-ethylhexyl 3,5,5-trimethylhexanoate, 2-ethylhexyl 2-ethylhexanoate, 2-hexyldecyl stearate, 2-octyldecyl palmitate, oleyl oleate, oleyl erucate, erucyl oleate, erucyl erucate and synthetic or natural mixtures of such esters), fats, waxes and other natural and synthetic fatty substances, preferably esters of fatty acids with alcohols of low C number (e.g. with isopropanol, propylene glycol or glycerol) or esters of fatty alcohols with alkanoic acids of low C number or with fatty acids, alkyl benzoates (e.g. mixtures of n-dodecyl, n-tridecyl, n-tetradecyl and n-pentadecyl benzoate) and cyclic or linear silicone oils (e.g. dimethylpolysiloxanes, diethyl polysiloxanes, diphenylpolysiloxanes and mixed forms thereof).

Nurturing substances which are outstandingly suitable for combination with the formulations according to the invention also include the following:
- waxes, e.g. candelilla wax or carnauba wax,
- ceramides, these being understood as meaning N-acylsphingosines (fatty acid amides of sphingosine) or synthetic analogues of such lipids (so-called pseudoceramides), which markedly improve the water retention capacity of the stratum corneum,
- phospholipids, for example soya lecithin, egg lecithin and kephalins,
- petrolatum and paraffin and silicone oils, the latter including, inter alia, dialkylsiloxanes and alkylarylsiloxanes, such as dimethylpolysiloxane and methylphenylpolysiloxane, and their alkoxylated and quaternized derivatives.

An aqueous phase of a formulation according to the invention can advantageously comprise alcohols, diols or polyols of low C number, and their ethers, preferably ethanol, isopropanol, propylene glycol, glycerol, ethylene glycol, ethylene glycol monoethyl or monobutyl ether, propylene glycol monomethyl, monoethyl or monobutyl ether, diethylene glycol monomethyl or monoethyl ether and analogous products, and alcohols of low C number, e.g. ethanol, isopropanol, 1,2-propanediol and glycerol, and especially one or more thickeners which can advantageously be selected from the group comprising silicon dioxide, aluminium silicates, polysaccharides or derivatives thereof, e.g. hyaluronic acid, xanthan gum and hydroxypropyl methyl cellulose, and particularly advantageously from the group comprising polyacrylates, preferably a polyacrylate from the group comprising so-called carbopols, e.g. carbopols of types 980, 981, 1382, 2984 and 5984, each individually or in combination.

Mixtures to be used according to the invention that are in the form of an emulsion advantageously comprise one or more emulsifiers. O/W emulsifiers can advantageously be selected e.g. from the group comprising polyethoxylated, polypropoxylated or polyethoxylated and polypropoxylated products, for example:
- fatty alcohol ethoxylates,
- ethoxylated wool wax alcohols,
- polyethylene glycol ethers of the general formula

   R-O-(-CH₂-CH₂-O-)ₙ-R',
- fatty acid ethoxylates of the general formula R-COO-(-CH₂-CH₂-O-)ₙ-H,
- etherified fatty acid ethoxylates of the general formula R-COO-(-CH₂-CH₂-O-)ₙ-R',
- esterified fatty acid ethoxylates of the general formula R-COO-(-CH₂-CH₂-O-)ₙ-C(O)-R',
- polyethylene glycol glycerol fatty acid esters,
- ethoxylated sorbitan esters,
- cholesterol ethoxylates,
- ethoxylated triglycerides,
- alkyl ether carboxylic acids of the general formula

   R-COO-(-CH₂-CH₂-O-)ₙ-OOH,

   n being a number from 5 to 30,
- polyethoxylated sorbitol fatty acid esters,
- alkyl ether sulfates of the general formula

   R-O-(-CH₂-CH₂-O-)ₙ-SO₃-H,
- fatty alcohol propoxylates of the general formula

   R-O-(-CH₂-CH(CH₃)-O-)ₙ-H,
- polypropylene glycol ethers of the general formula

   R-O-(-CH₂-CH(CH₃)-O-)ₙ-R',
- propoxylated wool wax alcohols,
- etherified fatty acid propoxylates

   R-COO-(-CH₂-CH(CH₃)-O-)ₙ-R',
- esterified fatty acid propoxylates of the general formula R-COO-(-CH₂-CH(CH₃)-O-)ₙ-C(O)-R',
- fatty acid propoxylates of the general formula

   R-COO-(-CH₂-CH(CH₃)-O-)ₙH,
- polypropylene glycol glycerol fatty acid esters,
- propoxylated sorbitan esters,
- cholesterol propoxylates,
- propoxylated triglycerides,
- alkyl ether carboxylic acids of the general formula

   R-O-(-CH₂-CH(CH₃)-O-)ₙ-CH₂-COOH,
- alkyl ether sulfates or the corresponding acids of the general formula R-O-(-CH₂-CH(CH₃)-O-)ₙ-SO₃-H,
- fatty alcohol ethoxylates/propoxylates of the general formula R-O-Xₙ-Yₘ-H,
- polypropylene glycol ethers of the general formula

   R-O-Xₙ-Yₘ-R',
- etherified fatty acid propoxylates of the general formula R-COO-Xₙ-Yₘ-R',
- fatty acid ethoxylates/propoxylates of the general formula

   R-COO-Xₙ-Yₘ-H.

According to the invention, the polyethoxylated, polypropoxylated or polyethoxylated and polypropoxylated O/W emulsifiers used are particularly advantageously selected from the group of substances with HLB values of 11 - 18, and very particularly advantageously from those with HLB values of 14.5 - 15.5, if they contain saturated radicals R and R'. If the O/W emulsifiers contain unsaturated radicals R and/or R', or if isoalkyl derivatives are present, the preferred HLB value of such emulsifiers can also be lower or higher.

The fatty alcohol ethoxylates are advantageously selected from the group comprising ethoxylated stearyl alcohols, cetyl alcohols and cetylstearyl alcohols (cetearyl alcohols). The following are particularly preferred:
polyethylene glycol (13) stearyl ether (steareth-13), polyethylene glycol (14) stearyl - ether (steareth-14), polyethylene glycol (15) stearyl ether (steareth-15), polyethylene glycol (16) stearyl ether (steareth-16), polyethylene glycol (17) stearyl ether (steareth-17), polyethylene glycol (18) stearyl ether (steareth-18), polyethylene glycol (19) stearyl ether (steareth-19), polyethylene glycol (20) stearyl ether (steareth-20), polyethylene glycol (12) isostearyl ether (isosteareth-12), polyethylene glycol (13) isostearyl ether (isosteareth-13), polyethylene glycol (14) isostearyl ether (isosteareth-14), polyethylene glycol (15) isostearyl ether (isosteareth-15), polyethylene glycol (16) isostearyl ether (isosteareth-16), polyethylene glycol (17) isostearyl ether (isosteareth-17), polyethylene glycol (18) isostearyl ether (isosteareth-18), polyethylene glycol (19) isostearyl ether (isosteareth-19), polyethylene glycol (20) isostearyl ether (isosteareth-20), polyethylene glycol (13) cetyl ether (ceteth-13), polyethylene glycol (14) cetyl ether (ceteth-14), polyethylene glycol (15) cetyl ether (ceteth-15), polyethylene glycol (16) cetyl ether (ceteth-16), polyethylene glycol (17) cetyl ether (ceteth-17), polyethylene glycol (18) cetyl ether (ceteth-18), polyethylene glycol (19) cetyl ether (ceteth-19), polyethylene glycol (20) cetyl ether (ceteth-20), polyethylene glycol (13) isocetyl ether (isoceteth-13), polyethylene glycol (14) isocetyl ether (isoceteth-14), polyethylene glycol (15) isocetyl ether (isoceteth-15), polyethylene glycol (16) isocetyl ether (isoceteth-16), polyethylene glycol (17) isocetyl ether (isoceteth-17), polyethylene glycol (18) isocetyl ether (isoceteth-18), polyethylene glycol (19) isocetyl ether (isoceteth-19), polyethylene glycol (20) isocetyl ether (isoceteth-20), polyethylene glycol (12) oleyl ether (oleth-12), polyethylene glycol (13) oleyl ether (oleth-13), polyethylene glycol (14) oleyl ether (oleth-14), polyethylene glycol (15) oleyl ether (oleth-15), polyethylene glycol (12) lauryl ether (laureth-12), polyethylene glycol (12) isolauryl ether (isolaureth-12), polyethylene glycol (13) cetylstearyl ether (ceteareth-13), polyethylene glycol (14) cetylstearyl ether (ceteareth-14), polyethylene glycol (15) cetylstearyl ether (ceteareth-15), polyethylene glycol (16) cetylstearyl ether (ceteareth-16), polyethylene glycol (17) cetylstearyl ether (ceteareth-17), polyethylene glycol (18) cetylstearyl ether (ceteareth-18), polyethylene glycol (19) cetylstearyl ether (ceteareth-19), polyethylene glycol (20) cetylstearyl ether (ceteareth-20).

The fatty acid ethoxylates can also advantageously be selected from the following group:
polyethylene glycol (20) stearate, polyethylene glycol (21) stearate, polyethylene glycol - (22) stearate, polyethylene glycol (23) stearate, polyethylene glycol (24) stearate, polyethylene glycol (25) stearate, polyethylene glycol (12) isostearate, polyethylene glycol (13) isostearate, polyethylene glycol (14) isostearate, polyethylene glycol (15) isostearate, polyethylene glycol (16) isostearate, polyethylene glycol (17) isostearate, polyethylene glycol (18) isostearate, polyethylene glycol (19) isostearate, polyethylene glycol (20) isostearate, polyethylene glycol (21) isostearate, polyethylene glycol (22) isostearate, polyethylene glycol (23) isostearate, polyethylene glycol (24) isostearate, polyethylene glycol (25) isostearate, polyethylene glycol (12) oleate, polyethylene glycol (13) oleate, polyethylene glycol (14) oleate, polyethylene glycol (15) oleate, polyethylene glycol (16) oleate, polyethylene glycol (17) oleate, polyethylene glycol (18) oleate, polyethylene glycol (19) oleate, polyethylene glycol (20) oleate.

Sodium laureth-11 carboxylate can advantageously be used as an ethoxylated alkyl ether carboxylic acid or a salt thereof. Sodium laureth-1-4 sulfate can advantageously be used as an alkyl ether sulfate. Polyethylene glycol (30) cholesteryl ether can advantageously be used as an ethoxylated cholesterol derivative. Polyethylene glycol (25) soya sterol has also proved valuable.

Polyethylene glycol (60) evening primrose glycerides can advantageously be used as ethoxylated triglycerides.

It is also advantageous to select the polyethylene glycol glycerol fatty acid esters from the group comprising polyethylene glycol (20) glyceryllaurate, polyethylene glycol (21) glyceryllaurate, polyethylene glycol (22) glyceryllaurate, polyethylene glycol (23) glyceryllaurate, polyethylene glycol (6) glycerylcaprylate/caprate, polyethylene glycol (20) glyceryloleate, polyethylene glycol (20) glycerylisostearate and polyethylene glycol (18) glyceryloleate/cocoate.

It is likewise favourable to select the sorbitan esters from the group comprising polyethylene glycol (20) sorbitan monolaurate, polyethylene glycol (20) sorbitan monostearate, polyethylene glycol (20) sorbitan mono isostearate, polyethylene glycol (20) sorbitan monopalmitate and polyethylene glycol (20) sorbitan monooleate.

The following can be used as advantageous W/O emulsifiers: fatty alcohols having 8 to 30 carbon atoms, monoglyceryl esters of saturated and/or unsaturated, branched and/or unbranched alkanecarboxylic acids with a chain length of 8 to 24 C atoms, especially 12 to 18 C atoms, diglyceryl esters of saturated and/or unsaturated, branched and/or unbranched alkanecarboxylic acids with a chain length of 8 to 24 C atoms, especially 12 to 18 C atoms, monoglyceryl ethers of saturated and/or unsaturated, branched and/or unbranched alcohols with a chain length of 8 to 24 C atoms, especially 12 to 18 C atoms, diglyceryl ethers of saturated and/or unsaturated, branched and/or unbranched alcohols with a chain length of 8 to 24 C atoms, especially 12 to 18 C atoms, propylene glycol esters of saturated and/or unsaturated, branched and/or unbranched alkanecarboxylic acids with a chain length of 8 to 24 C atoms, especially 12 to 18 C atoms, and sorbitan esters of saturated and/or unsaturated, branched and/or unbranched alkanecarboxylic acids with a chain length of 8 to 24 C atoms, especially 12 to 18 C atoms.

Particularly advantageous W/O emulsifiers are glyceryl monostearate, glyceryl monoisostearate, glyceryl monomyristate, glyceryl monooleate, diglyceryl monostearate, diglyceryl monoisostearate, propylene glycol monostearate, propylene glycol mono - isostearate, propylene glycol monocaprylate, propylene glycol monolaurate, sorbitan monoisostearate, sorbitan monolaurate, sorbitan monocaprylate, sorbitan mono-isooleate, sucrose distearate, cetyl alcohol, stearyl alcohol, arachidyl alcohol, behenyl alcohol, isobehenyl alcohol, selachyl alcohol, chimyl alcohol, polyethylene glycol (2) stearyl ether (steareth-2), glyceryl monolaurate, glyceryl monocaprate and glyceryl monocaprylate.

Mixtures to be used according to the invention (e.g. a topical cosmetic formulation) advantageously contain cooling agents. The following may be mentioned as examples of cooling agents: I-menthol, d-menthol, racemic menthol, menthone glyceryl acetal, menthyl lactate, substituted menthyl-3-carboxamides (e.g. menthyl-3-carboxylic acid N-ethylamide), 2-isopropyl-N-2,3-trimethylbutanamide, substituted cyclohexanecarboxamides, 3-menthoxypropane-1,2-diol, 2-hydroxyethyl menthyl carbonate, 2-hydroxypropyl menthyl carbonate, N-acetylglycine menthyl ester, isopulegol, hydroxycarboxylic acid menthyl esters (e.g. menthyl 3-hydroxybutyrate), monomenthyl succinate, 2-mercaptocyclodecanone, menthyl 2-pyrrolidin-5-onecarboxylate, 2,3-dihydroxy-p-menthane, 3,3,5-trimethylcyclohexanone glyceryl ketal, 3-menthyl-3,6-dioxaalkanoates and -trioxaalkanoates, 3-menthyl methoxyacetate and icilin.

The formulations according to the invention (e.g. topical cosmetic formulations) also advantageously contain antimicrobial substances. Other active substances worthy of particular mention in addition to conventional preservatives, i.e. in addition to the large group of conventional antibiotics, are the products relevant to cosmetics, such as triclosan, climbazole, zinc pyrithione, ichthyol, octopirox (2-aminoethanol salt of 1-hydroxy-4-methyl-6-(2,4,4-trimethylpentyl)-2(1H)-pyridone), chitosan, farnesol, octyloxyglycerol, glyceryl monolaurate, arylalkyl alcohols, e.g. phenylethyl alcohol, 3-phenyl-1-propanol, veticol or muguet alcohol, and aliphatic diols, e.g. 1,2-decanediol, or combinations of said substances which are used, inter alia, to combat armpit odour, foot odour or scaling.

Aryl-substituted or aryloxy-substituted, unbranched or monoalkyl- or polyalkyl-branched, saturated or unsaturated
- fatty alcohols, aldehydes, acids and acid esters,
- alkanediols, dialdehydes, dicarboxylic acids and dicarboxylic acid esters
with chain lengths of C₂ to C₄₀ from a synthetic or natural source (e.g. from coconut fat, palm kernel fat, wool wax, lanolin).

Monohydroxy and oligohydroxy fatty acids with chain lengths of C₂ to C₂₄ (e.g. lactic acid, 2-hydroxypalmitic acid), their oligomers and/or polymers and vegetable and animal raw materials containing these.

Ethoxylated, propoxylated or mixed ethoxylated/ propoxylated cosmetic fatty alcohols, fatty acids and fatty acid esters with chain lengths of C₂ to C₄₀ and having 1 to 150 EO and/or PO units.

It is also possible to use so-called "natural" antibacterial substances, most of which are ethereal oils. Examples of typical antibacterially active oils are those of anise, lemon, orange, rosemary, wintergreen, clove, thyme, lavender, hops, citronella, wheat, lemongrass, cedarwood, cinnamon, geranium, sandalwood, violet, eucalyptus, peppermint, gum benzoin, basil and fennel, as well as Ocmea origanum, Hydastis carradensis, Berberidaceae daceae, Ratanhiae or Curcuma longa.

Examples of important antimicrobially active substances which can be found in ethereal oils are anethole, catechol, camphene, carvacrol, eugenol, eucalyptol, ferulic acid, farnesol, hinokitiol, tropolone, limonene, menthol, methyl salicylate, thymol, terpineol, verbenone, berberin, curcumin, caryophyllene oxide, nerolidol and geraniol.

It is also possible to use mixtures of said active systems or active substances, as well as active substance combinations containing these active substances.

The amount of active substances in the preparations is preferably 0.01 to 20 wt.% and particularly preferably 0.05 - 10 wt.%, based on the total weight of the preparations.

Furthermore, a mixture to be used according to the invention can also be combined with sweat-inhibiting substances (antiperspirants) and odour absorbers. The antiperspirants used are primarily aluminium salts such as aluminium chloride, aluminium chlorohydrate, nitrate, sulfate, acetate, etc., and also aluminium hydroxychlorides. In addition to these, however, it can also be advantageous to use zinc, magnesium and zirconium compounds. The following can also be used: a) protein-precipitating substances such as, inter alia, formaldehyde, glutaraldehyde, natural and synthetic tannins and trichloroacetic acid, which bring about a surface occlusion of the sweat glands, b) local anaesthetics (inter alia, dilute solutions of e.g. lidocaine, prilocaine or mixtures of such substances), which switch off the sympathetic supply to the sweat glands by blocking the peripheral nerve paths, c) zeolites of the X, A or Y type, which, in addition to reducing sweat secretion, also act as adsorbents of bad odours, and d) botulinum toxin (toxin of the bacterium *Chlostridium botulinum*), and other substances that block the release of the transmitter substance acetylcholine relevant to sweat secretion.

Examples of odour absorbers are the sheet silicates described in Offenlegungsschrift DE-P 40 09 347, especially montmorillonite, kaolinite, nontronite, saponite, hectorite, bentonite and smectite, and also e.g. zinc salts of ricinoleic acid. They also include deodorants, bactericidal or bacteriostatic deodorizing substances, e.g. hexachlorophene, 2,4,4'-trichloro-2'-hydroxydiphenyl ether (Irgasan), 1,6-di(4-chlorophenylbiguanido)hexane (chlorhexidine), 3,4,4'-trichlorocarbanilide, and the active agents described in Offenlegungsschriften DE-37 40 186, DE-39 38 140, DE-42 04 321, DE-42 29 707, DE-42 29 737, DE-42 37 081, DE-43 09 372 and DE-43 24 219, which contain cationic substances, e.g. quaternary ammonium salts, and odour absorbers, e.g. ^{®}Grillocin (combination of zinc ricinoleate and various additives) or triethyl citrate, optionally in combination with ion exchange resins.

The amount of deodorizing and/or antiperspirant substances in the mixtures is preferably 0.01 to 20 wt.% and particularly preferably 0.05 -10 wt.%, based on the total weight of the preparations.

In numerous cases, the mixtures to be used in the formulations according to the invention can also advantageously be combined with preservatives. It is preferable here to choose preservatives like benzoic acid and its esters and salts, propionic acid and its salts, salicylic acid and its salts, 2,4-hexadienoic acid (sorbic acid) and its salts, formaldehyde and paraformaldehyde, 2-hydroxydiphenyl ether and its salts, zinc 2-sulfidopyridine N-oxide, inorganic sulfites and bisulfites, sodium iodate, chlorobutanolum, 4-ethylmercury(II)-5-amino-1,3-bis(2-hydroxybenzoic acid) and its salts and esters, dehydroacetic acid, formic acid, 1,6-bis(4-amidino-2-bromophenoxy)-n-hexane and its salts, the sodium salt of ethylmercury(II)-thiosalicylic acid, phenylmercury and its salts, 10-undecylenic acid and its salts, 5-amino-1,3-bis(2-ethylhexyl)-5-methylhexahydropyrimidine, 5-bromo-5-nitro-1,3-dioxane, 2-bromo-2-nitro-1,3-propanediol, 2,4-dichlorobenzyl alcohol, N-(4-chlorophenyl)-N'-(3,4-dichlorophenyl)urea, 4-chloro-m-cresol, 2,4,4'-trichloro-2'-hydroxydiphenyl ether, 4-chloro-3,5-dimethylphenol, 1,1'-methylenebis(3-(1-hydroxymethyl-2,4-dioximidazolidin-5-yl)urea), poly(hexamethylenediguanide) hydrochloride, 2-phenoxyethanol, hexamethylenetetramine, 1-(3-chloroallyl)-3,5,7-triaza-1-azoniaadamantane chloride, 1-(4-chlorophenoxy)-1-(1H-imidazo)-1-yl)-3,3-dimethyl-2-butanone, 1,3-bis(hydroxymethyl)-5,5-dimethyl-2,4-imidazolidinedione, benzyl alcohol, octopirox, 1,2-dibromo-2,4-dicyanobutane, 2,2'-methylenebis(6-bromo-4-chlorophenol), bromochlorophene, mixture of 5-chloro-2-methyl-3(2H)-isothiazolinone and 2-methyl-3(2H)-isothiazolinone with magnesium chloride and magnesium nitrate, 2-benzyl-4-chlorophenol, 2-chloroacetamide, chlorhexidine, chlorhexidine acetate, chlorhexidine gluconate, chlorhexidine hydrochloride, 1-phenoxypropan-2-ol, N-alkyl(C₁₂-C₂₂)trimethylammonium bromide and chloride, 4,4-dimethyl-1,3-oxazolidine, N-hydroxymethyl-N-(1,3-di(hydroxymethyl)-2,5-dioxoimidazolidin-4-yl)-N'-hydroxymethylurea, 1,6-bis(4-amidinophenoxy)-n-hexane and its salts, glutaraldehyde, 5-ethyl-1-aza-3,7-dioxabicyclo(3.3.0)octane, 3-(4-chlorophenoxy)-1,2-propanediol, hyamines, alkyl(C₈-C₁₈)dimethylbenzylammonium chloride, alkyl(C₈-C₁₈)dimethylbenzylammonium bromide, alkyl(C₈-C₁₈)dimethylbenzylammonium saccharinate, benzyl hemiformal, 3-iodo-2-propynylbutyl carbamate or sodium hydroxymethylaminoacetate.

Formulations according to the invention, especially dermatological formulations, can also advantageously contain dyestuffs and/or coloured pigments, especially if they are to be used in the decorative cosmetics sector. The dyestuffs and coloured pigments can be selected from the appropriate positive list of the cosmetics regulations or from the EC list of cosmetic colourants. In most cases they are identical to the dyestuffs permitted for foods. Examples of advantageous coloured pigments are titanium dioxide, mica, iron oxides (e.g. Fe₂O₃, Fe₃O₄, FeO(OH)) and/or tin oxide. Examples of advantageous dyestuffs are carmine, Berlin blue, chromium oxide green, ultramarine blue and/or manganese violet. Mixtures of said active systems can also be used.

The cosmetic use of a formulation according to the invention for strengthening the barrier function of damaged or undamaged skin or damaged or undamaged hair is furthermore disclosed, but not claimed.

A therapeutic or cosmetic method of strengthening the barrier function of damaged or undamaged skin or damaged or undamaged hair, comprising the following steps:
- preparation of a formulation according to the invention (preferably in a preferred form, cf. above), and
- application of an effective amount of the mixture to the damaged or undamaged skin or damaged or undamaged hair is also furthermore disclosed, but not claimed.

For application, a sufficient amount of topical mixtures to be used according to the invention (formulations) is applied to the skin and/or hair in the manner conventionally used for cosmetics.

The invention further relates to the use of a mixture of (a) one or more ceramides and/or pseudoceramides with (b) (alpha-)bisabolol for the preparation of a formulation according to the invention.

In addition to the pseudoceramide to be used according to claim 1 and also set out below, it is preferable to use a pseudoceramide from the group comprising:
- hydroxyethylpalmityloxyhydroxypropylpalmitamide,
- cetyloxypropylglycerylmethoxypropylmyristamide,
- 1,3-bis(N-(2-hydroxypropyl)acylamino)-2-hydroxypropane, where acyl = lauryl, myristoyl, palmitoyl, isostearoyl or stearoyl, and
- N-acylhydroxy-L-hydroxyproline .alkyl ester, where acyl = lauryl, myristoyl, palmitoyl, isostearoyl or stearoyl, and alkyl = C12 - C20 alkyl, preferably unbranched, and mixtures thereof.

The pseudoceramide to be used in accordance with the present invention is an N-acylhydroxyamino acid ester of formula I: 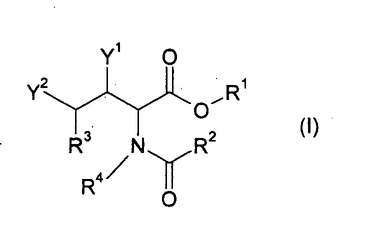 in which
R¹ is a linear, branched or cyclic alkyl or alkenyl group having 5 to 50 carbon atoms which is optionally substituted by one or more hydroxyl radicals,
R² is a linear or branched alkyl or alkenyl group having 1 to 49 carbon atoms which is optionally substituted by one or more hydroxyl radicals,
Y¹ and Y² independently of one another are hydrogen or hydroxyl, and
either R³ and R⁴ independently of one another are hydrogen or linear or branched alkyl or alkenyl groups having 1 to 10 carbon atoms,
or R³ and R⁴ together are an alkylene radical having 1 to 3 carbon atoms and form a 5-, 6- or 7-membered heterocyclic ring together with the chain between R³ and R⁴, said alkylene radical in turn optionally being substituted by 1 to 3 linear or branched alkyl or alkenyl groups or by 1 to 3 hydroxyl radicals.

R¹ is preferably a linear, branched or cyclic alkyl or alkenyl group having 5 to 24 carbon atoms which is optionally substituted by 1 to 6 hydroxyl radicals.

R² is preferably a linear or branched alkyl or alkenyl group having 2 to 23 carbon atoms which is optionally substituted by 1 to 6 hydroxyl radicals.

R² is particularly preferably a linear or branched alkyl or alkenyl group having 2 to 23 carbon atoms which is optionally substituted by 1 to 3 hydroxyl radicals.

One of the two groups Y¹ and Y² in formula (I) is preferably a hydroxyl radical and the other a hydrogen atom.

Preferably, R³ and R⁴ independently of one another are hydrogen or linear or branched alkyl or alkenyl groups having 1, 2, 3 or 4 carbon atoms, or R³ and R⁴ together are the alkylene radicals -CH₂-, -CH₂-CH₂-, -CH(OH)-, -CH(OH)-CH₂- or -CH₂-CH(OH)-.

Particularly preferably, R³ and R⁴ are hydrogen atoms and at the same time Y¹ and Y² independently of one another are hydrogen atoms or hydroxyl radicals, or R³ and R⁴ together are a -CH₂- or -CH(OH)- group and form a 5-membered heterocyclic ring together with the chain between R³ and R⁴, Y¹ and Y² at the same time being hydrogen atoms or hydroxyl radicals.

### A particularly advantageous form is one in which

R³ and R⁴ are hydrogen atoms and at the same time Y¹ is a hydroxyl radical and Y² a hydrogen atom (N-acylthreonine alkyl ester) or R³ and R⁴ together are a -CH₂- group and form a 5-membered heterocyclic ring together with the chain between R³ and R⁴, and one of the two radicals Y¹ and Y² is a hydroxyl radical (N-acylhydroxyproline ester).

R¹ in this case is preferably an unbranched alkyl or alkenyl radical having 5 to 24 carbon atoms and R² is an unbranched alkyl or alkenyl radical having 2 to 23 carbon atoms.

Other preferred forms of the invention can be found in the following Examples 1-10 and the attached Claims.

### Examples 1 -10: Formulations comprising (a) a ceramide or pseudoceramide and (b) alpha-bisabolol, together with (c) cholesterol or phytosterols and (d) fatty acids for strengthening the barrier function of the skin

Key to symbols in the Table below:
**1 = skin-lightening day cream O/W**
**2 = skin-soothing lotion with plant extracts O/W**
**3 = after-sun balm (not according to the present invention)**
**4 = body spray (not according to the present invention)**
**5 = sunscreen lotion (OIW), broad-band protection**
**6 = W/O night cream (not according to the present invention)**
**7 = shampoo**
**8 = self-bronzing cream (not according to the present invention)**
**9 = barrier repair cream O/W**
**10 = antiperspirant/deodorant roll-on (not according to the present invention)**

| **NAME OF RAW MATERIAL** | **INCI** | **WEIGHT** % | | | | | | | | | |
|---|---|---|---|---|---|---|---|---|---|---|---|
| **(MANUFACTURER)** | | **1** | **2** | **3** | **4** | **5** | **6** | **7** | **8** | **9** | **10** |
| **Pseudoceramides** | | | | | | | | | | | |
| **Pseudoceramide 176** | **N-(1-dodecanoyl)-4-hydroxy-L-proline 1-hexadecyl ester** | | | 0.1 | 0.2 | | | | 0.1 | | |
| **Pseudoceramide 391** | **N-(1-hexadecanoyl)-4-hydroxy-L-proline 1-hexadecyl ester** | 0.2 | 0.1 | | | 0.1 | 0.1 | 0.2 | | 0.5 | |
| **Ceramide PC104 (Pacific Corporation)** | **hydroxypropyl-bispalmi-tamide MEA** | | | | | 0.1 | | | | | |
| **Ceramide SL (Sino Lion)** | **hydroxyethyl- palmityl-oxyhydroxypropyl-palmitamide** | | | | | | 0.1 | | | | |
| **Aqua-Ceramide (Kao)** | **cetyloxypropyl- glyceryl-methoxypropyl- myristamide** | | 0.1 | | | | | | | | 0.1 |
| **Ceramides** | | | | | | | | | | | |
| **Ceramide 2 (Sederma)** | **ceramide 2** | 0.1 | | | | | | | | | |
| **Ceramide III (Cosmoferm)** | **ceramide 3** | | | | | 0.1 | | | | | |
| **Bisabolol** | | | | | | | | | | | |
| **(Alpha-)Bisabolol, natural (Symrise)** | **bisabolol** | | | 0.2 | | 0.3 | 0.2 | | | 0.5 | 0.1 |
| **Dragosantol (Symrise)** | **bisabolol** | 0.3 | 0.4 | | 0.2 | | | 0.3 | 0.3 | | |
| **Fatty acids** | | | | | | | | | | | |
| **Linoleic Acid** | **linoleic acid** | | | | | | 0.1 | | | 0.2 | |
| **Palmitic Acid** | **palmitic acid** | | 0.1 | | | | 0.1 | | | 0.2 | |
| **Stearic Acid** | **stearic acid** | | | | | | | | | 0.1 | |
| **Sterols** | | | | | | | | | | | |
| **Cholesterol (Croda)** | **cholesterol** | | 0.1 | | | | | | | 0.5 | |
| **Tamasterol (Tama Biochemicals)** | **phytosterol** | | | | | | 0.2 | | | | |
| **Other ingredients** | | | | | | | | | | | |
| **Abil 350 (Degussa-Goldschmidt)** | **dimethicone** | 0.5 | 2.0 | 1.0 | | | | | 0.5 | 0.5 | |
| **Allantoin (Merck)** | **allantoin** | | 0.2 | 0.1 | | | | | | | |
| **Aloe Vera Gel Concentrate 10/1 (Symrise)** | **water (aqua), Aloe barbadensis leaf juice** | | | 3.0 | | | 3.0 | | | | |
| **Alugel 34 TH (Baerlocher)** | **aluminium stearate** | | | | | | 1.0 | | | | |
| **Arbutin (Sabinsa)** | **β-arbutin** | 1.0 | | | | | | | | | |
| **Sodium Ascorbyl Phosphate (EMD Chemicals)** | **sodium ascorbyl phosphate** | 2.0 | | 1.0 | | | | | | | |
| **Butylene Glycol** | **butylene glycol** | | | 5.0 | | | | | | | |
| **Carbopol ETD 2050 (Noveon)** | **carbomer** | | | | | 0.2 | | | | | |
| **Carbopol Ultrez-10 (Noveon)** | **carbomer** | | 0.1 | | | | | | | | |
| **Cetiol OE (Cognis)** | **dicaprylyl ether** | | | 4.0 | | | | | | | |
| **Cetiol SB 45 (Cognis)** | **Butyrospermum parkii (shea butter)** | | | 1.0 | | | | | | | |
| **Citric Acid 10% sol.** | **citric acid** | | | | | | | 0.3 | | | |
| **Comperlan 100 (Cognis)** | **cocamide MEA** | | | | | | | 0.5 | | | |
| **Dihydroxyacetone (Merck)** | **dihydroxyacetone** | | | | | | | | 5.0 | | |
| **Dow Coming 246 Fluid (Dow Coming)** | **cyclohexasiloxane and cyclopentasiloxane** | | | | | 2.0 | | | | | |
| **Dow Coming 345 Fluid (Dow Coming)** | **cyclomethicone** | | | | 0.5 | | | | | | |
| **D-Panthenol (BASF)** | **panthenol** | | | 1.0 | | | | | | | |
| **Dracorin CE (Symrise)** | **glyceryl stearate- citrate** | 5.0 | | | | | | | 5.0 | 1.5 | |
| **Dracorin GMS (Symrise)** | **glycerol stearate** | | 2.0 | | | | | | | 2.0 | |
| **Dracorin GOC (Symrise)** | **glyceryl oleate- citrate, caprylic/capric triglyceride** | | | | 2.0 | | | | | | |
| **Drago-Beta-Glucan (Symrise)** | **water (aqua), butylene glycol, glycerol, Avena sativa (oat), kernel extract** | 0.3 | | | | | | | | | |
| **Dragocid Liquid (Symrise)** | **phenoxyethanol, methylparaben, ethylparaben, butylparaben, propylparaben, isobutylparaben** | | 0.8 | 0.7 | | 0.7 | 0.8 | | | 0.8 | |
| **Dragoderm (Symrise)** | **glycerol, Triticum vulgare (wheat) gluten, water (aqua)** | | | | | | | 2.0 | | | |
| **Drago-Oat-Active (Symrise)** | **water (aqua), butylene glycol, Avena sativa (oat) kernel extract** | | | | 1.0 | | | | | | |
| **Dragosan W/O Liquid (Symrise)** | **polyglyceryl-3-polyricinoleate, sorbitan isostearate** | | | | | | 1.0 | | | | |
| **Dragosan W/O P (Symrise)** | **sorbitan isostearate, hydrogenated castor oil, ceresin, beeswax (Cera alba)** | | | | | | 6.0 | | | | |
| **Dragoxat EH (Symrise)** | **ethylhexyl ethylhexanoate** | 3.0 | 3.0 | | 4.0 | | | | 3.0 | | |
| **Dragoxat 89 (Symrise)** | **ethylhexyl ethylisononanoate** | | | | | | | | | 2.0 | |
| **EDETA B Pulver (BASF)** | **tetrasodium EDTA** | | | | | | | 0.1 | | | |
| **EDETA DB (BASF)** | **disodium EDTA** | | | | | 0.1 | | | 0.1 | | |
| **Emulsiphos (Symrise)** | **potassium cetyl- phosphate, hydrogenated palm glycerides** | | 2.0 | | | 1.5 | | | | 2.0 | |
| **Ethanol 96%** | **ethanol** | | | | | | | | 2.0 | | 30.0 |
| **Extrapone Camomile GW (Symrise)** | **glycerol, water (aqua), Chamomilla recutita (matricaria) flower extract** | | 0.5 | | | | | | | | |
| **Extrapone Grüner Tee GW (Symrise)** | **glycerol, water (aqua), Camellia sinensis leaf extract** | | 0.2 | | | | | | | | |
| **Extrapone Witch Hazel Distillate colorless (Symrise)** | **propylene glycol, Hamamelis virginiana (witch hazel) water, water (aqua), Hamamelis virginiana (witch hazel) extract** | | | | | | 1.0 | | | | |
| **Extrapone Rosemary GW (Symrise)** | **glycerol, water (aqua), Rosmarinus officinalis (rosemary) leaf extract** | | 0.3 | | | | | | | 0.5 | |
| **Famesol (Symrise)** | **farnesol** | | | | | | | | | | 0.5 |
| **Frescolat ML crist. (Symrise)** | **menthyl lactate** | | | 0.8 | | | | | | | |
| **Genapol LRO liquid (Cognis)** | **sodium laureth sulfate** | | | | | | | 37.0 | | | |
| **Givobio GZN (Seppic)** | **zinc gluconate** | | | | | | | | | 0.5 | |
| **Glycerol 85%** | **glycerol** | 3.0 | 2.0 | 4.0 | | 4.7 | 2.0 | | 1.5 | 3.0 | |
| **Hydrolite-5 (Symrise)** | **pentylene glycol** | | | | 5.0 | | | | 3.5 | | |
| **Hydroviton (Symrise)** | **water, glycerol, sodium lactate, TEA lactate, serine, lactic acid, urea, sorbitol, sodium chloride, lauryl diethylenediamino-glycine, lauryl aminopropylglycine, allantoin** | | | | | | | | | 1.0 | |
| **Irgasan DP 300 (Ciba Geigy)** | **triclosan** | | | | | | | | | | 0.3 |
| **Isodragol (Symrise)** | **triisononanoin** | | 2.0 | | | | | | | 3.0 | |
| **Isofol 16 (Sasol)** | **hexyldecanol** | | 5.0 | | 2.0 | | 1.0 | | | | |
| **Isopropyl palmitate (Symrise)** | **isopropyl palmitate** | 4.0 | | | | | | | 4.0 | | |
| **Karion F (Merck)** | **sorbitol** | | | | | | 2.0 | | | | |
| **Keltrol RD (CP-Kelco)** | **xanthan gum** | 0.2 | 0.1 | | | | | | | | |
| **Keltrol T (Danby-Chemie)** | **xanthan gum** | | | | | 0.2 | | | 0.3 | | |
| **Kojic acid (Cosmetochem)** | **kojic acid** | 1.0 | | | | | | | | | |
| **Lanette 16 (Cognis)** | **cetyl alcohol** | 1.0 | | | | | | | 1.0 | | |
| **Lanette O (Cognis)** | **cetearyl alcohol** | | 3.0 | | | 1.0 | | | | 2.0 | |
| **Lara Care A-200 (Rahn)** | **galactoarabinan** | | | 0.3 | | | | | | | |
| **Magnesium Chloride (Merck)** | **magnesium chloride** | | | | | | 0.7 | | | | |
| **Merquat 550 (Ondeo Nalco)** | **polyquaternium-7** | | | | | | | 0.5 | | | |
| **NaOH 10% sol.** | **sodium hydroxide** | | | | | | | | | 0.3 | |
| **Naringin (Exquim)** | **4',5,7-trihydroxy-flavone-7-O-neohesperidoside** | | | | | | | 0.5 | 2.0 | | |
| **Sodium benzoate** | **sodium benzoate** | | | | | | | 0.5 | | | |
| **Natrosol 250 HHR (Aqualon)** | **hydroxyethyl cellulose** | | | | | | | | | | 0.3 |
| **Neo Heliopan 357 (Symrise)** | **butylmethoxydibenzoylmethane** | | | | | 1.0 | | | | | |
| **Neo Heliopan AP (Symrise) (10% as sodium salt)** | **disodium phenyl- dibenzimidazole- tetrasulfonate** | | | | | 10 | | | | | |
| **Neo Heliopan AV (Symrise)** | **ethylhexyl methoxycinnamate** | | | | | 3.0 | | | | | |
| **Neo Heliopan Hydro (Symrise) (15% as sodium salt)** | **phenyl-benzimidazolesulfonic acid** | | | | | 6.7 | | | | | |
| **Neo Heliopan MBC (Symrise)** | **4-methyl-benzylidene-camphor** | | | | | 1.5 | | | | | |
| **Neo Heliopan OS (Symrise)** | **ethylhexyl salicylate** | | | | | 5.0 | | | | | |
| **Neutral Oil** | **caprylic/capric triglyceride** | 6.0 | | | 4.0 | 2.0 | | | 6.0 | 10.0 | |
| **Oxynex 2004 (Merck)** | **BHT** | | | | | | 0.1 | | | | |
| **Paraffin oil 5 grade E (Parafluid)** | **paraffinum liquidum** | | | | 4.0 | | | | | | |
| **PCL Liquid 100 (Symrise)** | **cetearyl ethylhexanoate** | 3.0 | 5.0 | | 7.0 | | | | | | |
| **PCL Solid (Symrise)** | **stearyl heptanoate, stearyl caprylate** | | 2.0 | | | | | | | | |
| **PCL-Liquid (Symrise)** | **cetearyl ethylhexanoate, isopropyl myristate** | | | | | | 12.0 | | 3.0 | | |
| **Pemulen TR-2 (Noveon)** | **acrylates/C10-30 alkyl acrylate crosspolymer** | | | 0.3 | 0.2 | | | | | | |
| **Propylene Glycol-1,2 99P GC** | **propylene glycol** | | 5.0 | | | | | | | | |
| **Retinyl Palmitate in Oil (DSM Nutritional Products)** | **retinyl palmitate** | | | | | | 0.2 | | | | |
| **Sepigel 305** | **polyacrylamide, C13-14 isoparaffin, laureth-7** | | | | | | | | 1.0 | | |
| **Sodium Chloride** | **sodium chloride** | | | | | | | 1.0 | | | |
| **Sodium Hydroxide (10% sol.)** | **sodium hydroxide** | | 0.3 | 0.6 | 0.4 | | | | | | |
| **Solubilizer 611674 (Symrise)** | **PEG-40 hydrogenated castor oil, trideceth-9, water (aqua)** | | | | | | | | | | 2.0 |
| **Sun Flower Oil (Wagner)** | **Helianthus annuus (sunflower) seed oil** | | | | | | 5.0 | | | | |
| **Sweet Almond Oil (Wagner)** | **Prunus dulcis** | | | | | | 5.0 | | | | |
| **SymMatrix (Symrise)** | **maltodextrin, Rubus fruticosus (blackberry) leaf extract** | | 0.1 | | | 0.3 | 1.0 | | | | |
| **Symdiol 68 (Symrise)** | **1,2-hexanediol, caprylyl glycol** | 0.5 | | | | | | | | | |
| **Symrise Fragrance** | **fragrance** | 0.3 | 0.3 | 0.3 | 0.2 | 0.4 | 0.4 | 0.5 | 0.3 | 0.3 | 1.0 |
| **Tamasterol (Tama Biochemicals)** | **phytosterols** | | | | | | | | | 0.3 | |
| **Tego Betain L7 (Degussa)** | **cocamidopropyl betaine** | | | | | | | 6.0 | | | |
| **Tegosoft PC 31 (Degussa)** | | | | | | | | | | 0.3 | |
| **Tegosoft TN (Degussa)** | **C12-15 alkyl benzoate** | | | 5.0 | | 5.0 | | | | | |
| **Triethanolamine, 99%** | **triethanolamine** | | | | | 0.5 | | | | | |
| **Tocopherol Acetate (DSM Nutritional Products)** | **tocopheryl acetate** | | | 0.5 | | 0.5 | 3.0 | | | 0.3 | |
| **Zirkonal L 450 (BK Giulini)** | **aluminium zirconium pentachlorohyd rate (40% aqueous solution)** | | | | | | | | | | 37.0 |
| **Water, demineralized** | **water (aqua)** | ad 100 | ad 100 | ad 100 | ad 100 | ad 100 | ad 100 | ad 100 | ad 100 | ad 100 | ad 100 |

## Claims

1. Cosmetic or pharmaceutical formulation that can be applied to the skin or hair, comprising:
(a) one or more pseudoceramides or ceramides and pseudoceramides wherein the pseudoceramide used is an N-acylhydroxyamino acid ester of formula I: 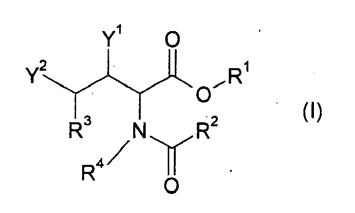 in which
R¹ is a linear, branched or cyclic alkyl or alkenyl group having 5 to 50 carbon atoms which is optionally substituted by one or more hydroxyl radicals,
R² is a linear or branched alkyl or alkenyl group having 1 to 49 carbon atoms which is optionally substituted by one or more hydroxyl radicals,
Y¹ and Y² independently of one another are hydrogen or hydroxyl, and
either R³ and R⁴ independently of one another are hydrogen or linear or branched alkyl or alkenyl groups having 1 to 10 carbon atoms,
or R³ and R⁴ together are an alkylene radical having 1 to 3 carbon atoms and form a 5-, 6- or 7-membered heterocyclic ring together with the chain between R³ and R⁴, said alkylene radical in turn optionally being substituted by 1 to 3 linear or branched alkyl or alkenyl groups or by 1 to 3 hydroxyl radicals,
and
(b) (alpha-)bisabolol,
the proportion by weight of (alpha-)bisabolol in the formulation being equal to or greater than the total proportion by weight of ceramides and/or pseudoceramides, and the total amount of components (a) and (b) being sufficient to strengthen the barrier of damaged and/or undamaged skin.

2. Formulation according to Claim 1 also comprising:
(c) cholesterol and/or one or more phytosterols, and
(d) one or more fatty acids, preferably palmitic acid and/or stearic acid.

3. Formulation according to Claim 2 wherein components (a), (b), (c) and (d) are used in a weight ratio of (0.5 - 3):(1 - 5):(0.5 - 3):(0.5- 3).

4. Formulation according to one of the preceding claims wherein the proportion of component (a) in the formulation ranges from 0.01 to 20 wt.%, preferably from 0.01 to 10 wt.% and particularly preferably from 0.01 to 5 wt.%, and the proportion of component (b) in the formulation ranges from 0.001 to 20 wt.%, preferably from 0.01 to 10 wt.% and particularly preferably from 0.05 to 5 wt.%.

5. Use of a mixture of (a) one or more pseudoceramides or ceramides and pseudoceramides, wherein the pseudoceramide used is an N-acylhydroxyamino acid ester of formula I: 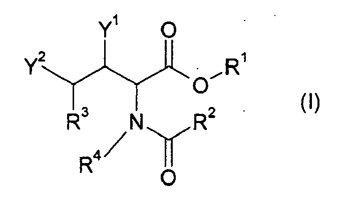 in which
R¹ is a linear, branched or cyclic alkyl or alkenyl group having 5 to 50 carbon atoms which is optionally substituted by one or more hydroxyl radicals,
R² is a linear or branched alkyl or alkenyl group having 1 to 49 carbon atoms which is optionally substituted by one or more hydroxyl radicals,
Y¹ and Y² independently of one another are hydrogen or hydroxyl, and
either R³ and R⁴ independently of one another are hydrogen or linear or branched alkyl or alkenyl groups having 1 to 10 carbon atoms,
or R³ and R⁴ together are an alkylene radical having 1 to 3 carbon atoms and form a 5-, 6- or 7-membered heterocyclic ring together with the chain between R³ and R⁴, said alkylene radical in turn optionally being substituted by 1 to 3 linear or branched alkyl or alkenyl groups or by 1 to 3 hydroxyl radicals,
with (b) (alpha-) bisabolol for the preparation of a formulation according to one of Claims 1-4.

6. Formulation or use according to one of Claims 1 - 5 in which R¹ is a linear, branched or cyclic alkyl or alkenyl group having 5 to 24 carbon atoms which is optionally substituted by 1 to 6 hydroxyl radicals.

7. Formulation or use according to Claim 6 in which R² is a linear or branched alkyl or alkenyl group having 2 to 23 carbon atoms which is optionally substituted by 1 to 6 hydroxyl radicals.

8. Formulation or use according to Claim 7 in which R² is a linear or branched alkyl or alkenyl group having 2 to 23 carbon atoms which is optionally substituted by 1 to 3 hydroxyl radicals.

9. Formulation or use according to one of the preceding Claims 6 - 8 in which one of the two groups Y¹ and Y² is a hydroxyl radical and the other is a hydrogen atom.

10. Formulation or use according to one of the preceding Claims 6 - 9 in which
R³ and R⁴ independently of one another are hydrogen or linear or branched alkyl or alkenyl groups having 1, 2, 3 or 4 carbon atoms, or
R³ and R⁴ together are the alkylene radicals -CH₂-,
-CH₂-CH₂-, -CH(OH)-, -CH(OH)-CH₂- or -CH₂-CH(OH)-.

11. Formulation or use according to one of the preceding Claims 6 - 10 in which
R³ and R⁴ are hydrogen atoms and at the same time Y¹ and Y² independently of one another are hydrogen atoms or hydroxyl radicals, or
R³ and R⁴ together are a -CH₂- or -CH(OH)- group and form a 5-membered heterocyclic ring together with the chain between R³ and R⁴, and at the same time Y¹ and Y² are hydrogen atoms or hydroxyl radicals.

12. Formulation or use according to Claim 11 in which
R³ and R⁴ are hydrogen atoms and at the same time Y¹ is a hydroxyl radical and Y² is a hydrogen atom (N-acylthreonine alkyl ester), or
R³ and R⁴ together are a -CH₂- group and form a 5-membered heterocyclic ring together with the chain between R³ and R⁴, and one of the two radicals Y¹ and Y² is a hydroxyl radical (N-acylhydroxyproline ester).

13. Formulation or use according to Claim 12 in which R¹ is an unbranched alkyl or alkenyl radical having 5 to 24 carbon atoms and R² is an unbranched alkyl or alkenyl radical having 2 to 23 carbon atoms.

## Patentansprüche

1. Auf die Haut oder das Haar auftragbare kosmetische oder pharmazeutische Formulierung, umfassend:
(a) ein oder mehrere Pseudoceramide oder Ceramide und Pseudoceramide, wobei das eingesetzte Pseudoceramid ein N-Acyl-hydroxyaminosäureester der Formel I ist: 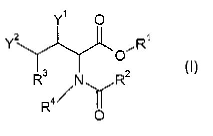 worin
R¹ eine lineare, verzweigte oder cyclische Alkyl- oder Alkenylgruppe mit 5 bis 50 Kohlenstoffatomen ist, die gegebenenfalls mit einem oder mehreren Hydroxylresten substituiert ist,
R² eine lineare oder verzweigte Alkyl- oder Alkenylgruppe mit 1 bis 49 Kohlenstoffatomen ist, die gegebenenfalls mit einem oder mehreren Hydroxylresten substituiert ist,
Y¹ und Y² unabhängig voneinander Wasserstoff oder Hydroxyl sind, und
R³ und R⁴ entweder unabhängig voneinander für Wasserstoff oder lineare oder verzweigte Alkyl- oder Alkenylgruppen mit 1 bis 10 Kohlenstoffatomen stehen oder
R³ und R⁴ zusammen einen Alkylenrest mit 1 bis 3 Kohlenstoffatomen darstellen und zusammen mit der Kette zwischen R³ und R⁴ einen 5-, 6- oder 7-gliedrigen heterocyclischen Ring bilden, wobei dieser Alkylenrest seinerseits gegebenenfalls durch 1 bis 3 lineare oder verzweigte Alkyl- oder Alkenylgruppen oder durch 1 bis 3 Hydroxylreste substituiert ist,
und
(b) (alpha-)Bisabolol,
wobei der Gewichtsanteil an alpha-Bisabolol in der Formulierung gleich oder größer ist als der Gesamt-Gewichtsanteil an Ceramiden und/oder Pseudoceramiden und
wobei die Gesamtmenge der Komponenten (a) und (b) ausreicht, die Hautbarriere geschädigter und/oder ungeschädigter Haut zu stärken.

2. Formulierung nach Anspruch 1, weiter umfassend
(c) Cholesterin und/oder ein oder mehrere Phytosterole, und
(d) eine oder mehrere Fettsäuren, vorzugsweise Palmitinsäure und/oder Stearinsäure.

3. Formulierung nach Anspruch 2,
wobei die Komponenten (a), (b), (c) und (d) im Gewichtsverhältnis (0.5 - 3) : (1 - 5) : (0.5 - 3) (0.5 - 3) eingesetzt werden.

4. Formulierung nach einem der vorangehenden Ansprüche,
wobei
der Anteil der Komponente (a) an der Formulierung im Bereich von 0,01 - 20 Gew.-% liegt, vorzugsweise im Bereich von 0,01 - 10 Gew.%, besonders bevorzugt im Bereich von 0,01 - 5 Gew.-%
und
der Anteil der Komponente (b) an der Formulierung im Bereich von 0,001 - 20 Gew.-% liegt, vorzugsweise im Bereich von 0,01 - 10 Gew.-%, besonders bevorzugt im Bereich von 0,05 - 5 Gew.-%.

5. Verwendung einer Mischung aus (a) einem oder mehreren Pseudoceramiden oder Ceramiden und Pseudoceramiden, wobei das eingesetzte Pseudoceramid ein N-Acyl-hydroxyaminosäureester der Formel I ist: 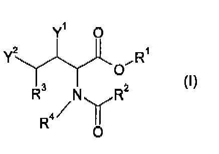 worin
R¹ eine lineare, verzweigte oder cyclische Alkyl- oder Alkenylgruppe mit 5 bis 50 Kohlenstoffatomen ist, die gegebenenfalls mit einem oder mehreren Hydroxylresten substituiert ist,
R eine lineare oder verzweigte Alkyl- oder Alkenylgruppe mit 1 bis 49 Kohlenstoffatomen ist, die gegebenenfalls mit einem oder mehreren Hydroxylresten substituiert ist,
Y¹ und Y² unabhängig voneinander Wasserstoff oder Hydroxyl sind, und
R³ und R⁴ entweder unabhängig voneinander für Wasserstoff oder lineare oder verzweigte Alkyl- oder Alkenylgruppen mit 1 bis 10 Kohlenstoffatomen stehen oder
R³ und R⁴ zusammen einen Alkylenrest mit 1 bis 3 Kohlenstoffatomen darstellen und zusammen mit der Kette zwischen R³ und R⁴ einen 5-, 6- oder 7-gliedrigen heterocyclischen Ring bilden, wobei dieser Alkylenrest seinerseits gegebenenfalls durch 1 bis 3 lineare oder verzweigte Alkyl- oder Alkenylgruppen oder durch 1 bis 3 Hydroxylreste substituiert ist,
mit (b) alpha-Bisabolol zur Herstellung einer Formulierung nach einem der Ansprüche 1-4.

6. Formulierung oder Verwendung nach einem der Ansprüche 1-5, wobei R¹ eine lineare, verzweigte oder cyclische Alkyl- oder Alkenylgruppe mit 5 bis 24 Kohlenstoffatomen ist, die gegebenenfalls mit 1 bis 6 Hydroxylresten substituiert ist.

7. Formulierung oder Verwendung nach Anspruch 6, wobei R² eine lineare oder verzweigte Alkyl- oder Alkenylgruppe mit 2 bis 23 Kohlenstoffatomen ist, die gegebenenfalls mit 1 bis 6 Hydroxylresten substituiert ist.

8. Formulierung oder Verwendung nach Anspruch 7, wobei R² eine lineare oder verzweigte Alkyl- oder Alkenylgruppe mit 2 bis 23 Kohlenstoffatomen ist, die gegebenenfalls mit 1 bis 3 Hydroxylresten substituiert ist.

9. Formulierung oder Verwendung nach einem der vorangehenden Ansprüche 6-8, wobei eine der beiden Gruppen Y¹ und Y² einen Hydroxylrest und die andere ein Wasserstoffatom bedeutet.

10. Formulierung oder Verwendung nach einem der vorangehenden Ansprüche 6-9, wobei
R¹ und R⁴ unabhängig voneinander für Wasserstoff oder lineare oder verzweigte Alkyl- oder Alkenylgruppen mit 1, 2, 3 oder 4 Kohlenstoffatomen stehen oder
R³ und R⁴ zusammen für die Alkylenreste -CH₂-, -CH₂-CH₂-, -CH(OH)-, -CH(OH)-CH₂- oder -CH₂-CH(OH)- stehen.

11. Formulierung oder Verwendung nach einem der vorangehenden Ansprüche 6-10, wobei
R³ und R⁴ Wasserstoffatome sind und gleichzeitig Y¹ und Y² unabhängig voneinander Wasserstoffatome oder Hydroxylreste sind, oder
R³ und R⁴ zusammen eine -CH₂- oder eine -CH(OH)--Gruppe darstellen und zusammen mit der Kette zwischen R³ und R⁴ einen 5-gliedrigen heterocyclischen Ring bilden und gleichzeitig Y¹ und Y² Wasserstoffatome oder Hydroxylreste sind.

12. Formulierung oder Verwendung nach Anspruch 11, wobei
R³ und R⁴ Wasserstoffatome sind und gleichzeitig Y¹ einen Hydroxylrest und Y² ein Wasserstoffatom darstellt (N-Acyl-threoninalkylester) oder
R³ und R⁴ zusammen eine -CH₂--Gruppe darstellen und zusammen mit der Kette zwischen R³ und R⁴ einen 5-gliedrigen heterocyclischen Ring bilden und einer der beiden Reste Y¹ und Y² einen Hydroxylrest darstellt (N-Acyl-hydroxyprolinester).

13. Formulierung oder Verwendung nach Anspruch 12, wobei R¹ einen unverzweigten Alkyl- oder Alkenylrest mit 5 bis 24 Kohlenstoffatomen und R² einen unverzweigten Alkyl- oder Alkenylrest mit 2 bis 23 Kohlenstoffatomen bedeutet.

## Revendications

1. Formulation cosmétique ou pharmaceutique qui peut être appliquée à la peau ou aux cheveux, comprenant :
(a) un ou plusieurs pseudocéramides ou céramides et pseudocéramides où le pseudocéramide utilisé est un ester de N-acylhydroxyaminoacide de formule I : 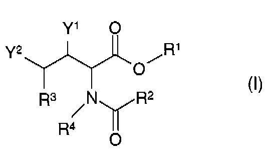 où
R¹ est un groupe alkyle ou alcényle linéaire, ramifié ou cyclique ayant 5 à 50 atomes de carbone qui est éventuellement substitué par un ou plusieurs radicaux hydroxyle,
R² est un groupe alkyle ou alcényle linéaire ou ramifié ayant 1 à 49 atomes de carbone qui est éventuellement substitué par un ou plusieurs radiaux hydroxyle,
Y² et Y² sont indépendamment l'un de l'autre l'hydrogène ou hydroxyle, et
R³ et R⁴ sont indépendamment l'un de l'autre l'hydrogène ou des groupes alkyle ou alcényle linéaires ou ramifiés ayant 1 à 10 atomes de carbone,
ou bien R³ et R⁴ sont ensemble un radical alkylène ayant 1 à 3 atomes de carbone et forment un cycle hétérocyclique à 5, 6 ou 7 chaînons avec la chaîne entre R³ et R⁴, ledit radical alkylène étant à son tour éventuellement substitué par 1 à 3 groupes alkyle ou alcényle linéaires ou ramifiés ou par 1 à 3 radicaux hydroxyle,
et
(b) de l'(alpha-)bisabolol,
la proportion en masse d'(alpha-)bisabolol dans la formulation étant égale ou supérieure à la proportion totale en masse de céramides et/ou pseudocéramides, et la quantité totale de composants (a) et (b) étant suffisante pour renforcer la barrière de la peau endommagée et/ou non endommagée.

2. Formulation selon la revendication 1 comprenant aussi :
(c) du cholestérol et/ou un ou plusieurs phytostérols, et
(d) un ou plusieurs acides gras, de préférence l'acide palmitique et/ou l'acide stéarique.

3. Formulation selon la revendication 2 où les composants (a), (b), (c) et (d) sont utilisés dans un rapport massique de (0,5 - 3):(1-5):(0,5 - 3):(0,5 - 3).

4. Formulation selon l'une des revendications précédentes où la proportion de composant (a) dans la formulation va de 0,01 à 20 % en masse, de préférence de 0,01 à 10 % en masse et de manière particulièrement préférable de 0,01 à 5 % en masse, et la proportion de composant (b) dans la formulation va de 0,001 à 20 % en masse, de préférence de 0,01 à 10 % en masse et de manière particulièrement préférable de 0,05 à 5 % en masse.

5. Utilisation d'un mélange de (a) un ou plusieurs pseudocéramides ou céramides et pseudocéramides où le pseudocéramide utilisé est un ester de N-acylhydroxyaminoacide de formule I : 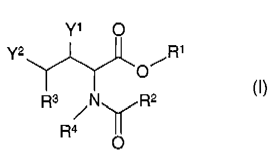 où
R¹ est un groupe alkyle ou alcényle linéaire, ramifié ou cyclique ayant 5 à 50 atomes de carbone qui est éventuellement substitué par un ou plusieurs radicaux hydroxyle,
R² est un groupe alkyle ou alcényle linéaire ou ramifié ayant 1 à 49 atomes de carbone qui est éventuellement substitué par un ou plusieurs radiaux hydroxyle,
Y¹ et Y² sont indépendamment l'un de l'autre l'hydrogène ou hydroxyle, et
R³ et R⁴ sont indépendamment l'un de l'autre l'hydrogène ou des groupes alkyle ou alcényle linéaires ou ramifiés ayant 1 à 10 atomes de carbone,
ou bien R³ et R⁴ sont ensemble un radical alkylène ayant 1 à 3 atomes de carbone et forment un cycle hétérocyclique à 5, 6 ou 7 chaînons avec la chaîne entre R³ et R⁴, ledit radical alkylène étant à son tour éventuellement substitué par 1 à 3 groupes alkyle ou alcényle linéaires ou ramifiés ou par 1 à 3 radicaux hydroxyle, et
(b) (alpha-)bisabol pour la préparation d'une formulation selon l'une des revendications 1 - 4.

6. Formulation ou utilisation selon l'une des revendications 1 - 5 où R¹ est un groupe alkyle ou alcényle linéaire, ramifié ou cyclique ayant 5 à 24 atomes de carbone qui est éventuellement substitué par 1 à 6 radicaux hydroxyle.

7. Formulation ou utilisation selon la revendication 6 où R² est un groupe alkyle ou alcényle linéaire ou ramifié ayant 2 à 23 atomes de carbone qui est éventuellement substitué par 1 à 6 radicaux hydroxyle.

8. Formulation ou utilisation selon la revendication 7 où R² est un groupe alkyle ou alcényle linéaire ou ramifié ayant 2 à 23 atomes de carbone qui est éventuellement substitué par 1 à 3 radicaux hydroxyle.

9. Formulation ou utilisation selon l'une des revendications 6 - 8 précédentes où l'un des deux groupes Y¹ et Y² est un radical hydroxyle et l'autre est un atome d'hydrogène.

10. Formulation ou utilisation selon l'une des revendications 6 - 9 précédentes où
R³ et R⁴ sont indépendamment l'un de l'autre l'hydrogène ou des groupes alkyle ou alcényle linéaires ou ramifiés ayant 1, 2, 3 ou 4 atomes de carbone, ou bien
R³ et R⁴ sont ensemble les radicaux alkylène -CH₂-, -CH₂-CH₂-, -CH(OH)-, -CH(OH)-CH₂- ou -CH₂-CH(OH)-.

11. Formulation ou utilisation selon l'une des revendications 6 - 10 précédentes où
R³ et R⁴ sont des atomes d'hydrogène et en même temps Y¹ et Y² sont indépendamment l'un de l'autre des atomes d'hydrogène ou des radicaux hydroxyle, ou
R³ et R⁴ sont ensemble un groupe -CH₂- ou -CH(OH)- et forment un cycle hétérocyclique à 5 chaînons avec la chaîne entre R³ et R⁴, et en même temps Y¹ et Y² sont des atomes d'hydrogène ou des radicaux hydroxyle.

12. Formulation ou utilisation selon la revendication 11 où
R³ et R⁴ sont des atomes d'hydrogène et en même temps Y¹ est un radical hydroxyle et Y² est un atome d'hydrogène (alkylester de N-acylthréonine), ou
R³ et R⁴ sont ensemble un groupe -CH₂- et forment un cycle hétérocyclique à 5 chaînons avec la chaîne entre R³ et R⁴, et l'un des deux radicaux Y¹ et Y² est un radical hydroxyle (ester de N-acylhydroxyproline).

13. Formulation ou utilisation selon la revendication 12 où R¹ est un radical alkyle ou alcényle non ramifié ayant 5 à 24 atomes de carbone et R² est un radical alkyle ou alcényle non ramifié ayant 2 à 23 atomes de carbone.
